# EUROPÄISCHE PATENTANMELDUNG

(11) **EP 2 025 674 A1**
(43) Veröffentlichungstag der Anmeldung: **18.02.2009**
(21) Anmeldenummer: 07291010.2
(22) Anmeldetag: 15.08.2007
(51) Int. Cl.: C07D 307/12, A61P 3/00

(54) **Substituierte Tetrahydronaphthaline, Verfahren zu ihrer Herstellung und ihre Verwendung als Arzneimittel**

(71) Anmelder: sanofi-aventis, 75013 Paris (FR)
(72) Erfinder: Schwink, Lothar, Dr., 65926 Frankfurt am Main (DE); Stengelin, Siegfried, Dr., 65926 Frankfurt am Main (DE); Gossel, Matthias, Dr., 65926 Frankfurt am Main (DE); Haack, Torsten, Dr., 65926 Frankfurt am Main (DE); Lennig, Petra, Dr., 65926 Frankfurt am Main (DE)
(74) Vertreter: Fischer, Hans-Jürgen

(57) **Zusammenfassung**

Die Erfindung befrifft substituierte Tetrahydronaphthaline der Formel I sowie deren physiologisch verträgliche Salze, deren Herstellung, Arzneimittel enthaltend mindestens ein erfindungsgemäßes substituiertes Tetrahydronaphthalin und die Verwendung der erfindungsgemäßen substituierten Tetrahydronaphthaline und deren Derivate als MCH-Antagonisten. 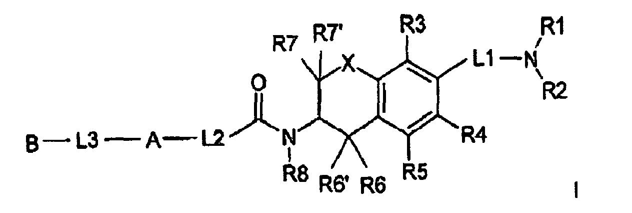 X O, C(R43)(R43');
B (C₁-C₆)-Alkyl, (C₁-C₄)-Alkoxy-(C₁-C₄)-alkyl, Hydroxy-(C₁-C₆)-alkyl, ein 3 bis 10-gliedriger mono-, bi- oder spirocyclischer nicht-aromatischer Ring, welcher 0 bis 3 Heteroatome beinhalten kann, ausgewählt aus der Gruppe Sauerstoff, Stickstoff und Schwefel, wobei das Ringsystem zusätzlich substituiert sein kann mit einem oder mehreren der folgenden Substituenten: F, CF₃, (C₁-C₆)-Alkyl, O-(C₁-C₈)-Alkyl, (C₁-C₄)-Alkoxy-(C₁-C₄)-alkyl, Hydroxy-(C₁-C₄)-alkyl, Oxo, CO(R64), Hydroxy;
wobei im Fall X=C(R43)(R43')
L3 C(R62)(R63)O, und
B ein 4 bis 10-gliedriger mono-, bi- oder spirocyclischer nicht-aromatischer Ring, welcher 1 bis 3 Heteroatome beinhaltet, ausgewählt aus der Gruppe Sauerstoff, Stickstoff und Schwefel, wobei das Ringsystem zusätzlich substituiert sein kann mit einem oder mehreren der folgenden Substituenten: F, CF₃, (C₁-C₆)-Alkyl, O-(C₁-C₈)-Alkyl, (C₁-C₄)-Alkoxy-(C₁-C₄)-alkyl, Hydroxy-(C₁-C₄)-alkyl, Oxo, CO(R64), Hydroxy;
bedeuten.

## Beschreibung

Die Erfindung betrifft substituierte Tetrahydronaphthaline und deren Derivate, sowie deren physiologisch verträgliche Salze und physiologisch funktionelle Derivate, deren Herstellung, Arzneimittel enthaltend mindestens ein erfindungsgemäßes substituiertes Tetrahydronaphthalin oder dessen Derivat und die Verwendung der erfindungsgemäßen substituierten Tetrahydronaphthaline und deren Derivate als Arzneimittel.

Es sind bereits den in der vorliegenden Anmeldung beschriebenen substituierten Tetrahydronaphthalinen und deren Derivaten in ihrer Gesamtstruktur ähnliche Verbindungen mit pharmakologischer Wirkung im Stand der Technik beschrieben, z. B. in der WO2002/064565 und der WO 2000/051970.
In WO2005/033063, US2005/0075324, US 2006/0247239 sowie in Meyers K. M. et al., Biorganic & Medicinal Chemistry Letters 17, 2007, 814-18; Meyers K. M. et al., Biorganic & Medicinal Chemistry Letters 17, 2007, 819-22; Mendez-Andino J.L. et al., Biorganic & Medicinal Chemistry Letters 15, 2007, 2092-2105 werden Amino-substituierte Tetrahydronaphthalin-Derivate mit MCH R1-antagonistischer Wirkung zur Behandlung von Obesitas offenbart.

Weitere Verbindungen mit MCH-antagonistischer Wirkung zur Behandlung der Obesitas sind im Stand der Technik beschrieben (Beispiele: WO2005047293, WO2004092181, WO2005103039, WO2004024702, WO2001021577, WO2003035624, WO2002089729, WO2002006245, WO2002002744, WO2002057233, WO2003045313, WO2003097047, WO2002010146, WO 2003087044).

Der Erfindung lag die Aufgabe zugrunde, Verbindungen zur Verfügung zu stellen, die eine Gewichtsreduktion bei Säugetieren bewirken und die zur Prävention und Behandlung von Obesitas und Diabetes sowie deren vielfältigen Folgeerkrankungen geeignet sind.

Überraschenderweise wurde eine Serie von Verbindungen gefunden, die die Aktivität von MCH-Rezeptoren modulieren. Insbesondere zeichnen sich die Verbindungen durch einen Antagonismus des MCH R1 aus.

Die Erfindung betrifft daher Verbindungen der Formel I, 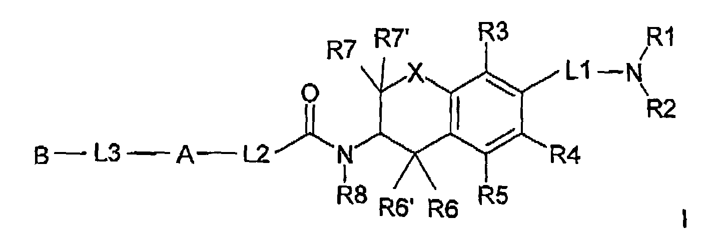 worin bedeuten
- R1, R2: unabhängig voneinander H, (C₁-C₈)-Alkyl, (C₁-C₄)-Alkoxy-(C₁-C₄)-alkyl, (C₃-C₈)-Alkenyl, (C₃-C₈)-Alkinyl, CO(R9), (C(R10)(R11))_{q}-R12, CO(C(R13)(R14))ᵣ-R15, CO-O(C₁-C₈)-Alkyl, CO(C(R13)(R14))ᵣ-N(R16)(R17);
oder
- R1 und R2: bilden zusammen mit dem Stickstoffatom, an das sie gebunden sind, einen 4 bis 10-gliedrigen mono-, bi- oder spirocyclischen Ring welcher außer dem Stickstoffatom 0 bis 3 zusätzliche Heteroatom beinhalten kann, ausgewählt aus der Gruppe Sauerstoff, Stickstoff und Schwefel, wobei das heterocyclische Ringsystem zusätzlich substituiert sein kann mit F, Cl, Br, CF₃, CN, (C₁-C₆)-Alkyl, (C₃-C₈)-Cycloalkyl, O-(C₁-C₈)-Alkyl, (C₁-C₄)-Alkoxy-(C₁-C₄)-alkyl, Hydroxy-(C₁-C₆)-alkyl, Oxo, CO(R18), CON(R19)(R20), Hydroxy, COO(R21), N(R22)CO(C₁-C₆)-Alkyl, N(R23)(R24) oder SO₂(C₁-C₆)-Alkyl;
- R10, R11: unabhängig voneinander H, (C₁-C₆)-Alkyl, OH;
- R9, R13, R14, R18, R19, R20, R21, R22, R23, R24,: unabhängig voneinander H, (C₁-C₆)-Alyl;
oder
- R23 und R24: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- q, r: unabhängig voneinander 0, 1, 2, 3, 4, 5, 6;
- R12, R15: unabhängig voneinander H, OH, F, O-(C₁-C₆)-Alkyl, S-(C₁-C₆)-Alkyl, CN, COO(R25), N(R26)CO(C₁-C₆)-Alkyl, N(R27)(R28), CON(R29)(R30), SO₂(C₁-C₆)-Alkyl, 3-12 gliedriger mono-, bi- oder spirocyclischer Ring, der ein oder mehrere Heteroatome aus der Gruppe N, O und S enthalten kann und der 3-12 gliedrige Ring weitere Substituenten wie F, Cl, Br, OH, CF₃, NO₂, CN, OCF₃, Oxo, O-(C₁-C₆)-Alkyl, (C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, N(R31)(R32), COO(R33), SO₂(C₁-C₆)-Alkyl und COOH enthalten kann;
- R25, R26, R27, R28, R29, R30, R31, R32, R33: unabhängig voneinander H, (C₁-C₈)-Alkyl;
oder
- R27 und R28, R29 und R30, R31 und R32: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- L1: C(R34)(R35), C(R36)(R37)C(R38)(R39), (C₃-C₆)-Cycloalkyl; optional kann R1 mit einem der Reste R34, R35, R36, R37, R38 oder R39 verbunden sein, so dass ein 5-6 gliedriger Ring entsteht;
- R34, R35, R36, R37, R38, R39: unabhängig voneinander H, (C₁-C₈)-Alkyl;
- R3, R4, R5: unabhängig voneinander H, F, Cl, Br, I, OH, CF₃, NO₂, CN, OCF₃, O-(C₁-C₆)-Alkyl, S-(C₁-C₆)-Alkyl, O-(C₁-C₄)-Alkoxy-(C₁-C₄)-alkyl, (C₁-C₆)-Alkyl, CON(R40)(R41), CO(R42);
- R40, R41, R42: unabhängig voneinander H, (C₁-C₈)-Alkyl;
oder
- R40 und R41: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- X: O, C(R43)(R43');
- R6, R6', R7, R7', R43, R43': unabhängig voneinander H, F, (C₁-C₈)-Alkyl, OH, O-(C₁-C₆)-Alkyl;
oder
- R6 und R6', oder R43 und R43': zusammen optional Oxo;
- R8: H, (C₁-C₈)-Alkyl;
- L2: Bindung, C(R44)(R45);
- R44, R45;: unabhängig voneinander H, (C₁-C₈)-Alkyl;
- A: 5-6 gliedriger aromatischer Ring, der bis zu 2 Heteroatome ausgewählt aus der Gruppe Stickstoff, Sauerstoff und Schwefel beinhalten kann, und substituiert sein kann mit einem oder mehreren der Substituenten H, F, Cl, Br, I, OH, CF₃, NO₂, CN, OCF₃, O-(C₁-C₆)-Alkyl, O-(C₁-C₄)-Alkoxy-(C₁-C₄)-alkyl, (C₁-C₆)-Alkyl, N(R54)(R55), SO₂-CH₃, CON(R56)(R57), N(R58)CO(R59), CO(R60);
- im Fall L2 =: Bindung kann C(O)NR8 mit einem ortho-ständigen Substituenten von A über eine Brücke enthaltend ein oder zwei Elemente aus der Gruppe Kohlenstoff und Stickstoff verbunden sein, so dass insgesamt ein 9 bis 10-gliedriger bicyclischer Ring entsteht;
- R54, R53, R56, R57, R58, R59, R60: unabhängig voneinander H, (C₁-C₈)-Alkyl;
oder
- R54 und R55, R56 und R57: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- L3: Bindung oder ein Linker mit 1 bis 4 Gliedern, wobei die Glieder ausgewählt sind aus der Gruppe bestehend aus O, S, SO₂, N(R61), CO, C(R62)(R63), C≡C, wobei sich ein chemisch sinnvoller Rest ergibt, und der Linker keine Gruppen O-CO oder COO aufweist;
- B: (C₁-C₆)-Alkyl, (C₁-C₄)-Alkoxy-(C₁-C₄)-alkyl, Hydroxy-(C₁-C₆)-alkyl, ein 3 bis 10-gliedriger mono-, bi- oder spirocyclischer nicht-aromatischer Ringe, welcher 0 bis 3 Heteroatome beinhalten kann, ausgewählt aus der Gruppe Sauerstoff, Stickstoff und Schwefel, wobei das Ringsystem zusätzlich substituiert sein kann mit einem oder mehreren der folgenden Substituenten: F, CF₃, (C₁-C₆)-Alkyl, O-(C₁-C₈)-Alkyl, (C₁-C₄)-Alkoxy-(C₁-C₄)-alkyl, Hydroxy-(C₁-C₄)-alkyl, Oxo, CO(R64), Hydroxy;
- R61, R62, R63, R64: unabhängig voneinander H, (C₁-C₈)-Alkyl;
wobei im Fall X = C(R43)(R43')
- L3: C(R62)(R63)O, und
- B: ein 4 bis 10-gliedriger mono-, bi- oder spirocyclischer nicht-aromatischer Ring, welcher 1 bis 3 Heteroatome beinhaltet, ausgewählt aus der Gruppe Sauerstoff, Stickstoff und Schwefel, wobei das Ringsystem zusätzlich substituiert sein kann mit einem oder mehreren der folgenden Substituenten: F, CF₃, (C₁-C₆)-Alkyl, O-(C₁-C₈)-Alkyl, (C₁-C₄)-Alkoxy-(C₁-C₄)-alkyl, Hydroxy-(C₁-C₄)-alkyl, Oxo, CO(R64), Hydroxy;
bedeuten.

Die Verbindungen der Formel I zeichnen sich dadurch aus, dass sie gegenüber strukturell ähnlichen Verbindungen mit MCH-antagonistischer Wirkung eine verbesserte Löslichkeit in wässrigen Medien (insbesondere in physiologisch relevanten Puffersystemen) bei gleichzeitiger hoher Aktivität aufweisen. Weiter zeichnen sich bevorzugte erfindungsgemäße Verbindungen durch eine geringe Blockade des hERG-Kanals aus. Des weiteren weisen bevorzugte erfindungsgemäße Verbindungen gegenüber Verbindungen des Standes der Technik eine verbesserte metabolische Stabilität auf.

Die Alkyl-, Alkenyl- und Alkinylreste in den Substituenten R1, R2, R3, R4, R5, R6, R6', R7, R7', R8, R9, R10, R11, R12, R13, R14, R15, R16, R17, R18, R19, R20, R21, R22, R23, R24, R25, R26, R27, R28, R29, R30, R31, R32, R33, R34, R35, R36, R37, R38, R39, R40, R41, R42, R43, R43', R44, R45, R54, R55, R56, R57, R58, R59, R60, R61, R62, R63, R64 können sowohl geradkettig, verzweigt und/oder optional substituiert sein mit Substituenten wie (C₁-C₄)-Alkoxy oder Halogen. Dies trifft auch zu, sofern die Alkyl-, Alkenyl- und Alkenylreste Teil einer anderen Gruppe sind, z.B. Teil einer Alkoxygruppe (wie (C₁-C₄)-Alkoxy-(C₁-C₄)-alkyl)). Geeignete Halogene sind Fluor, Chlor, Brom und Iod, bevorzugt Fluor, Chlor und Brom, besonders bevorzugt Fluor.

Beispiele für Alkylgruppen sind: Methyl, Ethyl, Propyl, Butyl, Pentyl, Hexyl, Heptyl und Octyl. Dabei sind sowohl die n-Isomere dieser Reste als auch verzweigte Isomere wie Isopropyl, Isobutyl, Isopentyl, sec-Butyl, tert-Butyl, Neopentyl, 3,3-Dimethylbutyl usw. mit umfasst. Sofern nicht anders beschrieben, beinhaltet der Begriff Alkyl darüber hinaus auch Alkylreste, die unsubstituiert oder gegebenenfalls mit einem oder mehreren weiteren Resten substituiert sind, beispielsweise mit 1, 2, 3 oder 4 identischen oder unterschiedlichen Resten, wie (C₁-C₄)-Alkoxy oder Halogen. Beispiele für mit Halogen substituierte Alkylgruppen sind fluorierte Alkylgruppen wie CF₃, CHF₂, CH₂F, 3-Fluorprop-1-yl, 2,2,1,1-Tetrafluorethyl. Dabei können die zusätzlichen Substituenten in jeder beliebigen Position des Alkylrestes auftreten. Bevorzugt sind die Alkylreste -soweit nicht anders definiert- unsubstituiert.

Unter Cycloalkyl ist im Sinne der vorliegenden Anmeldung Cycloalkyl sowie Cycloalkylalkyl- (Alkyl, das wiederum mit Cycloalkyl substituiert ist) zu verstehen, wobei Cycloalkyl mindestens 3 Kohlenstoffatome aufweist. Beispiele für Cycloalkylreste sind: Cyclopropyl, Cyclobutyl, Cyclopentyl, Cyclohexyl, Cycloheptyl, Cyclooctyl, Cyclononyl und Cyclodecyl. Gegebenenfalls kann es sich auch um polycyclische Ringsysteme handeln, wie Decalinyl, Norbornanyl, Bornanyl oder Adamantanyl. Die Cycloalkylreste können unsubstituiert oder gegebenenfalls mit einem oder mehreren weiteren Resten substituiert sein, wie vorstehend bei den Alkylresten beispielhaft aufgeführt. Bevorzugt sind die Cycloalkylreste -soweit nicht anders definiert- unsubstituiert.

Beispiele für Alkenyl- und Alkinylgruppen sind: Vinyl, 1-Propenyl, 2-Propenyl (Allyl), 2-Butenyl, 2-Methyl-2-propenyl, 3-Methyl-2-butenyl, Ethinyl, 2-Propinyl (Propargyl), 2-Butinyl oder 3-Butinyl.

Unter Cycloalkenyl sind im Sinne der vorliegenden Anmeldung Cycloalkenylresrte sowie Cycloalkenyl-Alkylreste (Alkyl, das mit Cycloalkenyl substituiert ist) zu verstehen, die mindestens drei Kohlenstoffatome enthalten. Beispiele für Cycloalkenyl sind: Cyclopentenyl, Cyclohexenyl, Cycloheptenyl und Cyclooctenyl.

Die Alkenylreste und Cycloalkenylreste können ein bis drei konjugierte oder nicht konjugierte Doppelbindungen (also auch Alk-dienyl- sowie Alk-trienylreste), vorzugsweise eine Doppelbindung in einer geraden oder verzweigten Kette aufweisen. Für Alkenylreste gilt das gleiche für die Dreifachbindungen. Die Alkenyl- und Alkenylreste können unsubstituiert oder gegebenenfalls mit einem oder mehreren weiteren Resten substituiert sein, wie vorstehend bei den Alkylresten beispielhaft aufgeführt. Bevorzugt sind die Alkenyl- und Alkinylreste -soweit nicht anders definiert- unsubstituiert.

Als Aryl werden in der vorliegenden Erfindung Reste bezeichnet, die von monocyclischen oder bicyclischen Aromaten abgeleitet sind, die keine Ringheteroatome enthalten. Sofern es sich nicht um monocyclische Systeme handelt, ist bei der Bezeichnung Aryl für den zweiten Ring auch die gesättigte Form (Perhydroform) oder die teilweise ungesättigte Form (beispielsweise die Dihydroform oder Tetrahydroform), sofern die jeweiligen Formen bekannt und stabil sind, möglich. Die Bezeichnung Aryl umfasst in der vorliegenden Erfindung beispielsweise auch bicyclische Reste, in denen sowohl beide Ringe aromatisch sind als auch bicyclische Reste, in denen nur ein Ring aromatisch ist. Beispiele für Aryl sind: Phenyl, Naphthyl, Indanyl, 1,2-Dihydronaphthenyl, 1,4-Dihydronaphthenyl, Indenyl oder 1,2,3,4-Tetrahydronaphthyl. Bevorzugt sind die Arylreste - soweit nicht anders definiert- unsubstituiert. Besonders bevorzugt ist Aryl Phenyl oder Naphthyl.

Unter Heteroarylresten sind Reste zu verstehen, die von monocyclischen oder bicyclischen Aromaten abgeleitet sind, die Ringbeteroatome, bevorzugt N, O oder S, enthalten. Im Übrigen gilt für die Heteroarylreste das bezüglich der Arylreste Aufgeführte.

Unter einem "Tricyclus" werden Strukturen mit 3 Ringen verstanden, die durch mehr als eine Bindung miteinander verbunden sind. Beispiele solcher Systeme sind kondensierte Systeme mit 3 Ringen und Spirocyclen mit ankondensiertem Ringsystem.

Unter einer polycyclischen Gruppe (bi-, tri- oder spirocyclisches Ringgerüst) ist im Sinne der vorliegenden Anmeldung eine Gruppe zu verstehen, die von Spiranen, kondensierten Ringsystemen oder Brücken-Ringsystemen abgeleitet ist. Die Spirane zeichnen sich dadurch aus, dass zwei Ringe nur ein Kohlenstoffatom gemeinsam aufweisen und die Ringebene der beiden Ringe senkrecht aufeinander stehen. Bei den kondensierten Ringsystemen sind zwei Ringe so miteinander verknüpft, dass sie zwei Atome gemeinsam aufweisen. Bei dieser Art der Verknüpfung handelt es sich um eine "*ortho*-Kondensation". Bei den Brücken-Ringsystemen handelt es sich um Ringsysteme, die eine Brücke aus Kohlenstoff- und/oder Heteroatomen zwischen zwei nicht benachbarten Atomen eines Rings aufweisen.

Unter einem "chemisch sinnvollen Rest" ist im Sinne der vorliegenden Erfindung ein Rest zu verstehen, der bei Raumtemperatur und Normaldruck stabil ist. Bevorzugt sind im Sinne der vorliegenden Erfindung unter einem "chemisch sinnvollen Rest" in der Definition der Gruppe A in den Verbindungen der Formel I Gruppen zu verstehen, die keine Heteroatom-Heteroatom-Bindungen zwischen den einzelnen Gliedern der Gruppen aufweisen.

Unter einem "nicht aromatischen" Ring ist im Sinne der vorliegenden Anmeldung bevorzugt ein Ring zu verstehen, der gesättigt oder teilweise ungesättigt ist. Dabei weist ein teilweise ungesättigter Ring gemäß der vorliegenden Anmeldung eine oder gegebenenfalls mehrere Doppelbindungen auf, wobei der teilweise ungesättigte Ring jedoch nicht aromatisch ist. Von dem Ausdruck "nicht aromatisch" sind im Sinne der vorliegenden Anmeldung auch "nicht heteroaromatische" Ringe umfasst.

Die Verbindungen der Formel I können ein oder mehrere Assymmetriezentren enthalten. Daher können die Verbindungen der Formel I in Form ihrer Racemate, Enantiomeren angereicherten Mischungen, reinen Enantiomere, Diastereomere und Diastereomer-Mischungen vorliegen. Die vorliegende Erfindung umfasst alle diese isomeren Formen der Verbindungen der Formel I. Diese Isomeren Formen können, wenn auch zum Teil nicht expressis verbis beschrieben, nach bekannnten Methoden erhalten werden.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze) und Salze von Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbiudung(en) gemäß Formel I" auf Verbindung(en) der Formel I, wie vorstehend beschrieben, sowie ihre Salze, Solvate und physiologisch funktionelle Derivate wie hierin beschrieben.

Können Reste oder Substituenten mehrfach in den Verbindungen der Formyl I auftreten, so können sie alle unabhängig voneinander die angegebenen Bedeutungen haben und gleich oder verschieden sein.

Bevorzugt weisen die Symbole in der Formel I unabhängig voneinander die folgenden Bedeutungen auf:
- R1, R2: unabhängig voneinander H, (C₁-C₈)-Alkyl, (C₁-C₄)-Alkoxy-(C₁-C₄)-alkyl, (C₃-C₈)-Alkenyl, (C₃-C₈)-Alkinyl, CO(R9), (C(R10)(R11))_{q}-R12, CO(C(R13)(RI4))ᵣ-R15, CO-O(C₁-C₈)-Alkyl, CO(C(R13)(R14))ᵣ-N(R16)(R17);
bevorzugt bedeutet R1:
H, (C₁-C₈)-Alkyl, (C(R10)(R11))_{q}-R12, (C₁-C₄)-Alkoxy-(C₁-C₄)-alkyl, (C₃-C₈)-Alkyl, (C₃-C₈)-Alkinyl, CO-(C₁-C₈)-Alkyl, CO-O(C₁-C₈)-Alkyl, CO(C(R13)(R14))ᵣN(R16)(R17);
bevorzugt bedeutet R2:
(C₁-C₈)-Alkyl, (C(R10)(R11))_{q}-R12, (C₁-C₄)-Alkoxy-(C₁-C₄)-alkyl, (C₃-C₈)-Alkenyl, (C₃-C₈)-Alkinyl;
oder
- R1 und R2: bilden zusammen mit dem Stickstoffatom, an das sie gebunden sind, einen 4 bis 10-gliedrigen mono-, bi- oder spirocyclischen Ring welcher außer dem Stickstoffatom 0 bis 3 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, Br, CF₃, CN, (C₁-C₆)-Alkyl, (C₃-C₈)-Cycloalkyl, O-(C₁-C₈)-Alkyl, (C₁-C₄)-Alkoxy-(C₁-C₄)-alkyl, Hydroxy-(C₁-C₆)-alkyl, Oxo, CO(R18), CON(R19)(R20), Hydroxy, COO(R21), N(R22)CO(C₁-C₆)-Alkyl, N(R23)(R24) oder SO₂(C₁-C₆)-Alkyl;
besonders bevorzugt bedeuten R1, R2:
(C₁-C₈)-Alkyl, (C(R10)(R11))_{q}-R12, (C₁-C₄)-Alkoxy-(C₁-C₄)-alkyl; oder R1 und R2 bilden zusammen mit dem Stickstoffatom, an das sie gebunden sind, einen 4 bis 10-gliedrigen mono-, bi- oder spirocyclischen Ring welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, Br, CF₃, (C₁-C₆)-Alkyl, O-(C₁-C₈)-Alkyl, (C₃-C₈)-Cycloalkyl, (C₁-C₄)-Alkoxy-(C₁-C₄)-alkyl, Hydroxy-(C₁-C₆)-alkyl, Oxo, CO(R18), Hydroxy, N(R22)CO(C₁-C₆)-Alkyl, oder SO₂(C₁-C₆)-Alkyl;
ganz besonders bevorzugt bilden R1 und R2 bilden zusammen mit dem Stickstoffatom, an das sie gebunden sind, einen 4 bis 10-gliedrigen mono-, bi- oder spirocyclischen Ring welcher ausser dem Stickstoffatom 0 bis 2 zusätzliche Heteroatome beinhalten karm, ausgewählt aus der Gruppe Sauerstoff, Stickstoff und Schwefel, wobei das heterocyclische Ringsystem zusätzlich substituiert sein kann mit F, Cl, Br, CF₃, (C₁-C₆)-Alkyl, O-(C₁-C₈)-Alkyl, (C₃-C₈)-Cycloalkyl, (C₁-C₄)-Alkoxy-(C₁-C₄)-alkyl, Hydroxy-(C₁-C₆)-alkyl, Oxo, CO(R18), Hydroxy, N(R22)CO(C₁-C₆)-Alkyl, oder SO₂(C₁-C₆)-Alkyl;
- R10, R11: unabhängig voneinander H, (C₁-C₆)-Alkyl, OH;
- R9, R13, R14, R18, R19, R20, R21, R22, R23, R24,: unabhängig voneinander H, (C₁-C₆)-Alkyl;
oder
- R23 und R24: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- q, r: unabhängig voneinander 0, 1, 2, 3, 4, 5, 6;
- R12, R15: unabhängig voneinander H, OH, F, O-(C₁-C₆)-Alkyl, S-(C₁-C₆)-Alkyl, CN, COO(R25), N(R26)CO(C₁-C₆)-Alkyl, N(R27)(R28), CON(R29)(R30), SO₂(C₁-C₆)-Alkyl, 3-12 gliedriger mono-, bi- oder spirocyclischer Ring, der ein oder mehrere Heteroatome aus der Gruppe N, O und S enthalten kann und der 3-12 gliedrige Ring weitere Substituenten wie F, Cl, Br, OH, CF₃, NO₂, CN, OCF₃, Oxo, O-(C₁-C₆)-Alkyl, (C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, N(R31)(R32), COO(R33), SO₂(C₁-C₆)-Alkyl und COOH enthalten kann;
bevorzugt H, OH, F, O-(C₁-C₆)-Alkyl, N(R26)CO(C₁-C₆)-Alkyl, SO₂(C₁-C₆)-Alkyl, 3-12 gliedriger mono-, bi- oder spirocyclischer Ring, der ein oder mehrere Heteroatome aus der Gruppe N, O und S enthalten kann und der 3-12 gliedrige Ring weitere Substituenten wie F, Cl, Br, OH, CF₃, NO₂, CN, OCF₃, Oxo, O-(C₁-C₆)-Alkyl, (C₁-C₄)-Alkoxy-(C₁-C₄)-alkyl, (C₁-C₆)-Alkyl, (C₂-C₆)-Alkenyl, (C₂-C₆)-Alkinyl, N(R31)(R32) und SO₂(C₁-C₆)-Alkyl enthalten kann;
- R25, R26, R27, R28, R29, R30, R31, R32, R33: unabhängig voneinander H, (C₁-C₈)-Alkyl;
oder
- R27 und R28, R29 und R30, R31 und R32: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- L1: C(R34)(R35), C(R36)(R37)C(R38)(R39), (C₃-C₆)-Cycloalkyl; bevorzugt C(R34)(R35);
optional kann R1 mit einem der Reste R34, R35, R36, R37, R38 oder R39 verbunden sein, so dass ein 5-6 gliedriger Ring entsteht;
- R34, R35, R36, R37, R38, R39: unabhängig voneinander H, (C₁-C₈)-Alkyl;
- R3, R4, R5: unabhängig voneinander H, F, Cl, Br, I, OH, CF₃, NO₂, CN, OCF₃, O-(C₁-C₆)-Alkyl, S-(C₁-C₆)-Alkyl, O-(C₁-C₄)-Alkoxy-(C₁-C₄)-alkyl, (C₁-C₆)-Alkyl, CON(R40)(R41), CO(R42);
bevorzugt unabhängig voneinander H, F, Cl, Br, CF₃, CN, OCF₃, O-(C₁-C₆)-Alkyl, (C₁-C₄)-Alkoxy-(C₁-C₄)-alkyl, (C₁-C₆)-Alkyl, CO(C₁-C₆)-Alkyl;
besonders bevorzugt unabhängig voneinander H, F, Cl, Br, CF₃, CN, OCF₃, O-(C₁-C₆)-Alkyl, (C₁-C₄)-Alkoxy-(C₁-C₄)-alkyl, (C₁-C₆)-Alkyl;
ganz besonders bevorzugt unabhängig voneinander H, F, Cl, O-(C₁-C₆)-Alkyl, (C₁-C₆)-Alkyl;
insbesonders besonders bevorzugt H;
wobei bevorzugt mindestens zwei, oder alle Reste R3, R4 und R5 H bedeuten;
- R40, R41, R42: unabhängig voneinander H, (C₁-C₈)-Alkyl;
oder
- R40 und R41: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- X: O, C(R43)(R43');
- R6, R6', R7, R7', R43, R43': unabhängig voneinander H, F, (C₁-C₈)-Alkyl, OH, O-(C₁-C₆)-Alkyl; bevorzugt H;
oder
- R6 und R6', oder R43 und R43': zusammen optional Oxo;
- R8: H, (C₁-C₈)-Alkyl;
- L2: Bindung, C(R44)(R45);
bevorzugt Bindung;
- R44, R45;: unabhängig voneinander H, (C₁-C₈)-Alkyl;
- A: 5-6 gliedriger aromatischer Ring, der bis zu 2 Heteroatome ausgewählt aus der Gruppe Stickstoff, Sauerstoff und Schwefel beinhalten kann, und substituiert sein kann mit einem oder mehreren der Substituenten H, F, Cl, Br, I, OH, CF₃, NO₂, CN, OCF₃, O-(C₁-C₆)-Alkyl, O-(C₁-C₄)-Akoxy-(C₁-C₄)-alkyl, (C₁-C₆)-Alkyl, N(R54)(R55), SO₂-CH₃, CON(R56)(R57), N(R58)CO(R59), CO(R60); bevorzugt H, F, Cl, Br, I, OH, CF₃, NO₂, CN, OCF₃, O-(C₁-C₆)-Alkyl, (C₁-C₄)-Alkoxy-(C₁-C₄)-alkyl, (C₁-C₆)-Alkyl; besonders bevorzugt H, F, Cl, Br, CF₃, CN, OCF₃, O-(C₁-C₆)-Alkyl, (C₁-C₄)-Alkoxy-(C₁-C₄)-alkyl, (C₁-C₆)-Alkyl; ganz besonders bevorzugt H, F, Cl, O-(C₁-C₆)-Alkyl, (C₁-C₆)-Alkyl; insbesondere ganz besonders bevorzugt H; bevorzugt ist der 5-6 gliedrige aromatische Ring ausgewählt aus der Gruppe bestehend aus 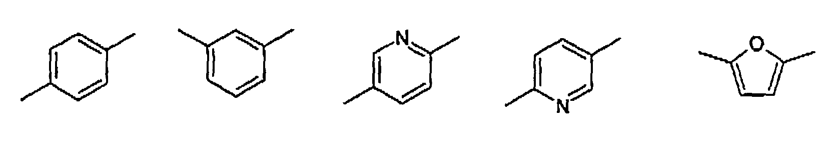 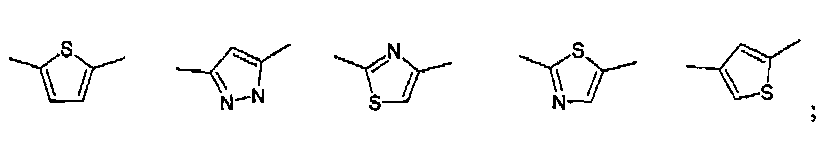 besonders bevorzugt 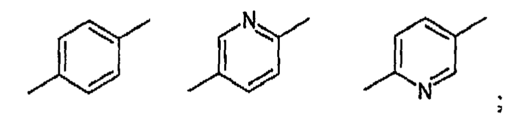
- im Fall L2 =: Bindung kann C(O)NR8 mit einem ortho-ständigen Substituenten von A über eine Brücke enthaltend ein oder zwei Elemente aus der Gruppe Kohlenstoff und Stickstoff verbunden sein, so dass insgesamt ein 9 bis 10-gliedriger bicyclischer Ring entsteht;
- R54, R55, R56, R57, R58, R59, R60: unabhängig voneinander H, (C₁-C₈)-Alkyl;
oder
- R54 und R55, R56 und R57: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- L3: Bindung oder ein Linker mit 1 bis 4 Gliedern, wobei die Glieder ausgewählt sind aus der Gruppe bestehend aus O, S, SO₂, N(R61), CO, C(R62)(R63), C≡C, wobei sich ein chemisch sinnvoller Rest ergibt, und der Linker keine Gruppen O-CO oder COO aufweist;
bevorzugt Bindung oder ein Linker mit 1 bis 4 Gliedern, wobei die Glieder ausgewählt sind aus der Gruppe bestehend aus O, N(R61), CO, C(R62)(R63), wobei sich ein chemisch sinnvoller Rest ergibt, und der Linker keine Gruppen O-CO oder COO aufweist;
besonders bevorzugt Bindung, O, C(R62)(R63)O;
- B: (C₁-C₆)-Alkyl, (C₁-C₄)-Alkoxy-(C₁-C₄)-alkyl, Hydroxy-(C₁-C₆)-alkyl, ein 3 bis 10-gliedriger mono-, bi- oder spirocyclischer nicht-aromatischer Ring, welcher 0 bis 3 Heteroatome beinhalten kann, ausgewählt aus der Gruppe Sauerstoff, Stickstoff und Schwefel, wobei das Ringsystem zusätzlich substituiert sein kann mit einem oder mehreren der folgenden Substituenten: F, CF₃, (C₁-C₆)-Alkyl, O-(C₁-C₈)-Alkyl, (C₁-C₄)-Alkoxy-(C₁-C₄)-alkyl, Hydroxy-(C₁-C₄)-alkyl, Oxo, CO(R64), Hydroxy;
bevorzugt ein 4 bis 6-gliedriger nicht-aromatischer Ring, welcher 1 bis 2 Sauerstoffatome beinhaltet, wobei das Ringsystem zusätzlich substituiert sein kann mit einem oder mehreren der folgenden Substituenten: F, (C₁-C₆)-Alkyl, O-(C₁-C₈)-Alkyl (C₁-C₄)-Alkoxy-(C₁-C₄)-alkyl, Oxo, Hydroxy bevorzugt (C₁-C₆)-Alkyl oder Hydroxy;
besonders bevorzugt das kombinierte Element B-L3
ausgewählt aus der Gruppe 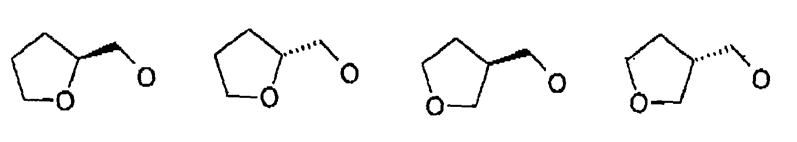 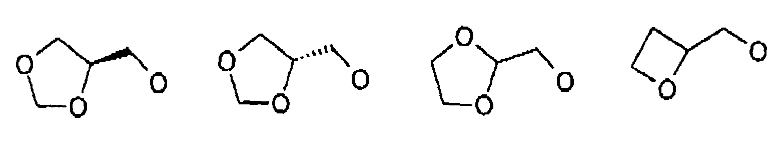 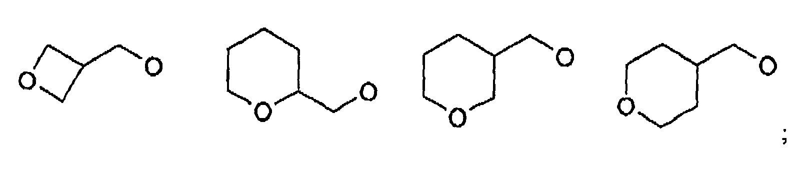
bevorzugt 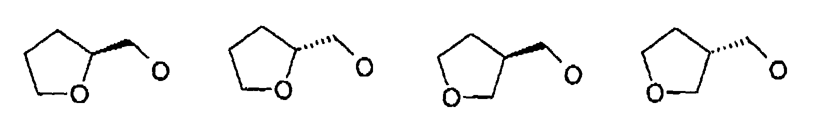 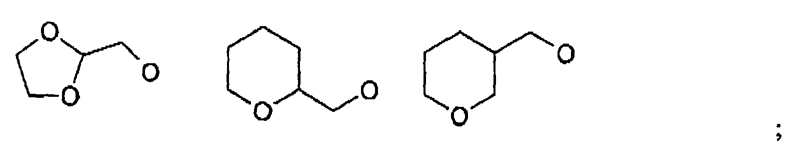 besonders bevorzugt 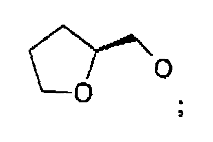
- R61, R62, R63, R64: unabhängig voneinander H, (C₁-C₈)-Alkyl;
wobei im Fall X = C(R43)(R43')
- L3: C(R62)(R63)O, und
- B: ein 4 bis 10-gliedriger mono-, bi- oder spirocyclischer nicht-aromatischer Ring, welcher 1 bis 3 Heteroatome beinhaltet, ausgewählt aus der Gruppe Sauerstoff, Stickstoff und Schwefel, wobei das Ringsystem zusätzlich substituiert sein kann mit einem oder mehreren der folgenden Substituenten: F, CF₃, (C₁-C₆)-Alkyl, O-(C₁-C₈)-Alkyl, (C₁-C₄)-Alkoxy-(C₁-C₄)-alkyl, Hydroxy-(C₁-C₄)-alkyl, Oxo, CO(R64), Hydroxy;
bedeuten.

Ein besonderer Aspekt der Erfindung sind die Verbindungen der Formel II 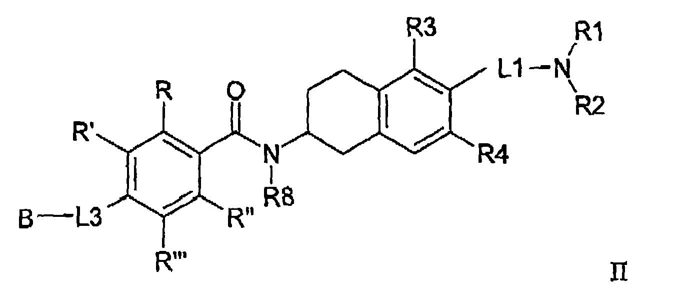 worin die Variablen R1, R2, L1, R3, R4 und R8 die oben angegebenen Bedeutungen haben und
- R, R', R'', R''': unabhängig voneinander H, F, Cl, Br, I, OH, CF₃, NO₂, CN, OCF₃, O-(C₁-C₆), Alkyl, O-(C₁-C₄)-Alkoxy-(C₁-C₄)-alkyl, (C₁-C₆)-Alkyl, N(R54)(R55), SO₂-CH₃, CON(R56)(R57), N(R58)CO(R59), CO(R60);
bevorzugt unabhängig voneinander H, F, Cl, Br, I, OH, CF₃, NO₂, CN, OCF₃, O-(C₁-C₆)-Alkyl, (C₁-C₄)-Alkoxy-(C₁-C₄)-alkyl, (C₁-C₆)-Alkyl;
besonders bevorzugt unabhängig voneinander H, F, Cl, Br, CF₃, CN, OCF₃, O-(C₁-C₆)-Alkyl, (C₁-C₄)-Alkoxy-(C₁-C₄)-alkyl, (C₁-C₆)-Alkyl;
ganz besonders bevorzugt unabhängig voneinander H, F, Cl, O-(C₁-C₆)-Alkyl, (C₁-C₆)-Alkyl;
insbesondere ganz besonders bevorzugt H;
- L3: CH₂O;
- B: 4 bis 6-gliedriger nicht-aromatischer Ring, welcher 1 bis 2 Sauerstoffatome beinhaltet, wobei das Ringsystem zusätzlich substituiert sein kann mit einem oder mehreren der folgenden Substituenten: F, (C₁-C₆)-Alkyl, O-(C₁-C₈)-Alkyl, (C₁-C₄)-Alkoxy-(C₁-C₄)-alkyl, Oxo, Hydroxy bevorzugt (C₁-C₆)-Alkyl oder Hydroxy;
- das kombinierte Element B-L3: bevorzugt ausgewählt aus der Gruppe 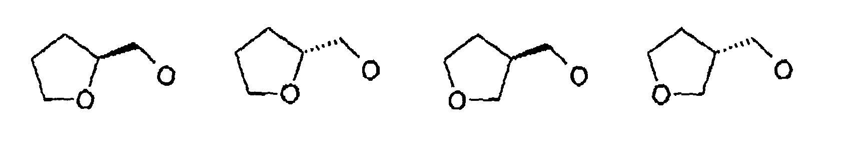 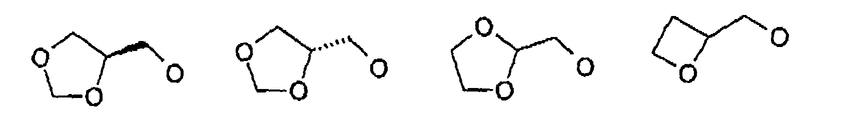 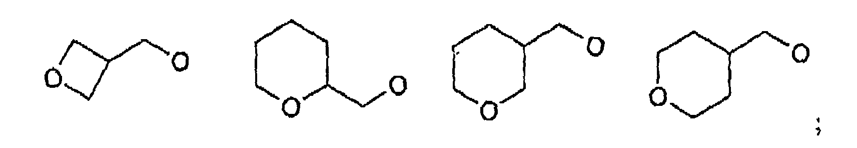 besonders bevorzugt 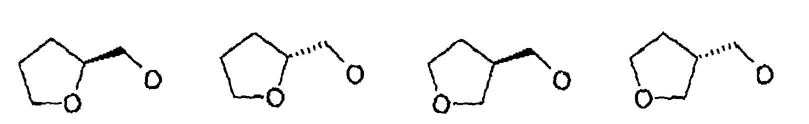 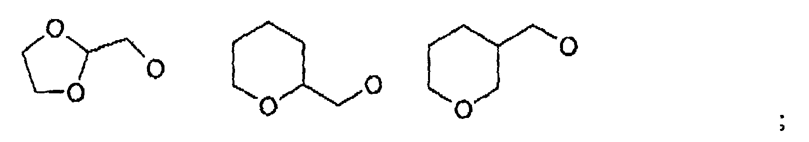 insbesondere bevorzugt 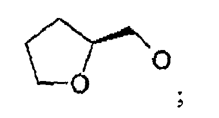
bedeuten.

In einem weiteren besonderen Aspekt betrifft die Erfindung Verbindungen der Formel III 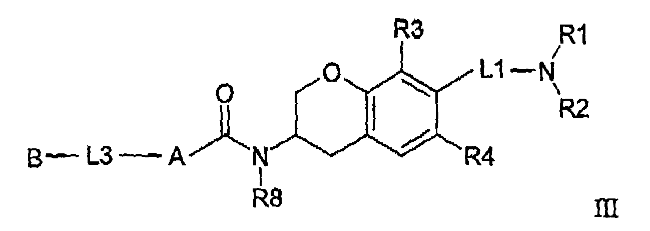 worin R1, R2, R3, R4, R8, A, L1, L3 und B die für Formel I angegebenen Bedeutungen haben.

In einem anderen besonderen Aspekt betrifft die Erfindung Verbindungen der Formel IV 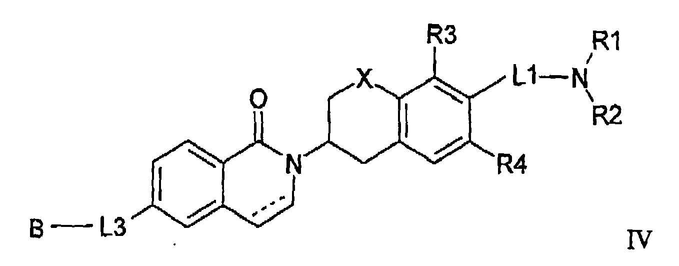 worin R1, R2, R3, R4, X, L1, L3 und B die für Formel I angegebenen Bedeutungen haben. Die unterbrochene Linie zeigt eine optionale Doppelbindung an, so dass sowohl Dihydroisochinolinone als auch Isochinolinone von der Formel IV umfasst werden.

Die erfindungsgemäßen Verbindungen der allgemeinen Formel I können analog zu dem Fachmann bekannten Verfahren hergestellt werden. Geeignete Verfahren zur Herstellung der erfindungsgemäßen Verbindungen der allgemeinen Formel 1 sind nachstehend beispielhaft erwähnt (siehe insbesondere Methoden A, B, C, D, E, F, G und Schemata 1 bis 3).

Bevorzugte Ausführungsformen der genannten Schritte ebenso wie die Herstellung der in den Schritten eingesetzten Ausgangssubstanzen sind dem Fachmann bekannt und in den genannten Schemata und Methoden sowie Beispielen beispielhaft erwähnt.

Diese Erfindung bezieht sich weiterhin auf die Verwendung von Verbindungen der Formel I und ihren pharmazeutischen Zusammensetzungen als MCH-Rezeptor-Liganden. Die erfindungsgemäßen MCH-Rezeptor-Liganden eignen sich insbesondere als Modulatoren der Aktivität des MCH R1.

Die Rolle von MCH in der Regulation des Energiehaushalts ist inzwischen gut dokumentiert (Qu, D. et al.; Nature 1996, 380, 243-7; Shimada, M. et al. Nature 1998, 396, 670-4; Chen, Y. et al. Endocrinology 2002, 143, 2469-77; Endocrinology 2003, 144, 4831-40; Übersichten: G. Hervieu, Expert Opin. Ther. Targets 2003, 7, 495-511; Shi, Y., Peptides 2004, 25, 1605-11; Pissios, P. et al. , Endocrine Rev. 2006, 27, 606-20).

Auch gibt es Hinweise, dass MCH-Antagonisten zentral bedingte Störungen wie z.B. Angstneurosen und Depressionen günstig beeinflussen können (Borowsky, B. et al.; Nature Medicine 2002, 8, 825-30; Übersichten: Hervieu, G., Expert Opin. Ther. Targets 2003, 7, 495-511; Chaki, S. et al., Drug Dev. Res. 2005, 65, 278-290; Dyck, B., Drug Dev. Res. 2005, 65, 291-300; Shimazaki, T., CNS Drugs 2006, 20, 801-11).

Besonders geeignet sind solche Verbindungen zur Behandlung und/oder Prävention von
1. Obesitas
2. Diabetes mellitus, insbesondere Typ 2 Diabetes einschließlich der Verhinderung der damit verbundenen Folgeerkrankungen.
   Besondere Aspekte sind dabei die
   - Hyperglykämie,
   - Verbesserung der Insulinresistenz,
   - Verbesserung der Glukose-Toleranz,
   - Schutz der β-Zellen der Bauchspeicheldrüse
   - Verhinderung makro- und mikrovaskulärer Erkrankungen
3. Dyslipidämien und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid-, hohe postprandiale Plasma-Triglycerid- Konzentrationen
   - niedrige HDL-Cholesterin Konzentration
4. Fettleber, insbesondere nichtalkoholische Fettleber und deren Verlaufsformen
   - Steatose
   - Steatohepatitis
   - Zirrhose
5. Verschiedene andere Zuständen, die mit dem Metabolischen Syndrom assoziert sein können, sind wie:
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myokardinfarkt, hypertensive Herzerkrankung oder Kardiomyopathie
6. Psychiatrische Indikationen wie
   - Depressionen
   - Angstzustände
   - Störungen des zirkadianen Rhythmus
   - Affektionsstörungen
   - Schizophrenie
   - Suchtkrankheiten

### Galenik

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,001 mg bis 100 mg (typischerweise von 0,01 mg bis 50 mg) pro Tag pro Kilogramm Körpergewicht, z.B. 0,1 -10 mg/kg/Tag. Eine intravenöse Dosis kann z.B. im Bereich von 0,001 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0.05 bis 1000 mg, typischerweise von 0,5 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge mindestens einer Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen *können, wie bereits erwähnt,* nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mitteln in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die mindestens eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen mindestens einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man mindestens eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Die Verbindungen der Formel I zeichnen sich durch günstige Wirkungen auf den Fettstoffwechsel aus, insbesondere sind sie zur Gewichtsreduktion und nach erfolgter Gewichtsreduktion zum Erhalt eines reduzierten Gewichtes bei Säugetieren und als Anorektika geeignet. Die Verbindungen zeichnen sich als selektive MCH1R-Antagonisten durch ihre geringe Toxizität, die geringe Beeinflussung von metabolisierenden Enzymen und ihre geringen Nebenwirkungen aus. Insbesondere zeichnen sich bevorzugte erfindungsgemäße Verbindungen durch eine geringe Blockade des hERG-Kanals aus. Weiterhin sind bevorzugte Verbindungen der Formel I in wässrigen Systemen merklich löslich und damit für eine pharmazeutische Entwicklung besonders geeignet. Auch wird die pharmakologische Wirkung in in vivo Prüfmodellen aus gut verträglichen Vehikeln heraus nach oraler Gabe erreicht.

Die Verbindungen können allein oder in Kombination mit weiteren gewichtsreduzierenden oder anorektischen Wirkstoffe eingesetzt werden. Solche weiteren anorektischen Wirkstoffe werden z.B. in der Roten Liste, Kapitel 01 unter Abmagerungsmittel/Appetitzügler genannt und können auch solche Wirkstoffe beinhalten, die den Energieumsatz des Organismus erhöhen und damit zu einer Gewichtsreduktion führen oder auch solche, welche den allgemeinen Metabolismus des Organismus so beeinflussen, dass eine erhöhte Kalorienzufuhr nicht zu einer Vergrößerung der Fettdepots und eine normale Kalorienzufuhr zu einer Verringerung der Fettdepots des Organismus führt. Die Verbindungen eignen sich zur Prophylaxe sowie insbesondere zur Behandlung von Übergewicht oder Obesitas. Die Verbindungen eignen sich weiterhin zur Prophylaxe sowie insbesondere zur Behandlung von Typ II Diabetes, der Arteriosklerose sowie zur Normalisierung des Lipidstoffwechsels und zur Behandlung des Bluthochdrucks.

### Kombinationen mit anderen Medikamenten

Die erfindungsgemäßen Verbindungen können allein oder in Kombination mit einer oder mehreren weiteren pharmakologisch wirksamen Substanzen verabreicht werden, die beispielsweise günstige Wirkungen auf Stoffwechselstörungen oder damit häufig assoziierte Erkrankungen haben. Solche Medikamente sind zum Beispiel
1. Blutzuckersenkende Medikamente, Antidiabetika,
2. Wirkstoffe zur Behandlung von Dyslipidemien,
3. Antiatherosklerotische Medikamente,
4. Antiadiposita,
5. Antiinflammatorische Wirkstoffe
6. Wirkstoffe zur Behandlung von malignen Tumoren
7. Antithrombotische Wirkstoffe
8. Wirkstoffe zur Behandlung von Bluthochdruck
9. Wirkstoffe zur Behandlung von Herzinsuffizienz sowie
10. Wirkstoffe zur Behandlung und/oder Prävention von Komplikationen, die von *Diabetes verursacht werden* oder mit Diabetes assoziiert sind.

Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

Beispielhaft seien die im Folgenden genannten Wirkstoffe als geeignet für Kombinationspräparate aufgeführt:

Alle Antidiabetika, die in der Roten Liste 2005, Kapitel 12 genannt sind; alle Abmagerungsmittel/Appetitzügler, die in der Roten Liste 2005, Kapitel 1 gennant sind; alle Lipidsenker, die in der Roten Liste 2005, Kapitel 58 genannt sind. Die meisten der nachfolgend aufgeführten Wirkstoffe sind in USP Dictionary of USAN and International Drug Names, US Pharmacopeia, Rockville 2001, offenbart.

Antidiabetika umfassen alle Insuline und Insulinderivate, wie z.B. Lantus^{®} (Insuline glargine, siehe www.lantus.com) oder Apidra ^{®} (Isuline glulisine, HMR 1964) oder solche, wie sie in WO2005005477 (Novo Nordisk) beschrieben sind, schnell wirkende Insuline (siehe US 6,221,633), inhalierbare Insuline, wie z. B. Exubera ^{®} oder orale Insuline, wie z. B. IN-105 (Nobex) oder Oral-lyn ™ (Generex Biotechnology), GLP-1-Derivate wie z.B. Exenatide, Liraglutide oder diejenigen, die in WO 98/08871, WO2005027978 von Novo Nordisk A/S, in WO 01/04156 von Zealand oder in WO 00/34331 von Beaufour-Ipsen offenbart wurden, Pramlintide Acetat (Symlin; Amylin Pharmaceuticals), sowie oral wirksame hypoglykämische Wirkstoffe.

Die oral wirksamen hypoglykämischen Wirkstoffe umfassen vorzugsweise
Sulfonylharnstoffe,
Biguanidine,
Meglitinide,
Oxadiazolidindione,
Thiazolidindione,
*Glukosidase-Inhibitoren*,
Hemmstoffe der Glykogenphosphorylase,
Glukagon-Antagonisten,
Glukokinaseaktivatoren,
Inhibitoren der Fructose-1,6-bisphosphatase
Modulatoren des Glukosetransporters-4 (GLUT4),
Inhibitoren der Glutamin-Fructose-6-Phosphat-Amidotransferase (GFAT),
GLP-1-Agonisten, Kaliumkanalöffner, wie z.B. diejenigen, die in WO 97/26265 und WO 99/03861 von Novo Nordisk A/S offenbart wurden,
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption, Hemmstoffe der 11β-HSD1,
Inhibitoren der Protein-Tyrosin-Phosphatase-1B (PTP1B),
Modulatoren des natrium-abhängigen Glukosetransporters 1 oder 2 (SGLT1, SGLT2), den Fettstoffwechsel verändernde Verbindungen wie antihyperlipidämische Wirkstoffe und antilipidämische Wirkstoffe,
Verbindungen, die die Nahrungsmitteleinnahme oder Nahrungsmittelaufnahme verringem, Verbindungen, die die Thermogenese erhöhen,
PPAR- und RXR-Modulatoren und
Wirkstoffe, die auf den ATP-abhängigen Kaliumkanal der Betazellen wirken.

Bei einer Ausführungsform der Erfindung wird mindestens eine Verbindung der Formel I in Kombination mit einem HMGCoA-Reduktase Inhibitor wie Simvastatin, Fluvastatin, Pravastatin, Lovastatin, Atorvastatin, Cerivastatin, Rosuvastatin, L-659699 verabreicht

Bei einer Ausführungsform der Erfindung wird mindestens eine Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692), MD-0727 (Microbia Inc., WO2005021497) oder mit Verbindungen, wie in WO2002066464 (Kotobuki Pharmaceutical Co. Ltd.) oder WO2005062824 (Merck & Co.) oder WO2005061451 und W02005061452 (AstraZeneca AB) beschrieben, verabreicht.

Bei einer Ausführungsform der Erfindung wird mindestens eine Verbindung der Formel I in Kombination mit einem PPAR gamma Agonisten, wie z.B. Rosiglitazon, Pioglitazon, JTT-501, GI 262570, R-483, CS-011 (Rivoglitazon) verabreicht.

Bei einer Ausführungsform der Erfindung wird mindestens eine Verbindung der Formel I in Kombination mit einem PPAR alpha Agonisten, wie z.B. GW9578, GW-590735, K-111, LY-674, KRP-101, DRF-10945 verabreicht

Bei einer Ausführungsform der Erfindung wird mindestens eine Verbindung der Formel I in Kombination mit einem gemischten PPAR alpha/gamma Agonisten, wie z.B. Muraglitazar, Tesaglitazar, Naveglitazar, LY-510929, ONO-5129, E-3030, AVE 8042, AVE 8134, AVE 0847, oder wie in PCT/US 00/11833, PCT/US 00/11490, DE10142734.4 oder in J.P.Berger et al., TRENDS in Pharmacological Sciences 28(5), 244-251, 2005 beschrieben, verabreicht.

Bei einer Ausführungsform der Erfindung wird mindestens eine Verbindung der Formel I in Kombination mit einem PPAR delta Agonisten, wie z.B. GW-501516 verabreicht.

Bei einer Ausführungsform wird mindestens eine Verbindung der Formel I in Kombination mit Metaglidasen oder mit MBX-2044 oder anderen partiellen PPAR gamma Agonisten/Antagonisten verabreicht.

Bei einer Ausführungsform der Erfindung wird mindestens eine Verbindung der Formel I in Kombination mit einem Fibrat, wie z.B. Fenofibrat, Clofibrat, Bezafibrat, verabreicht.

Bei einer Ausführungsform der Erfindung wird mindestens eine Verbindung der Formel I in Kombination mit einem MTP-Inhibitor, wie z.B. Implitapide, BMS-201038, R-103757 oder solchen wie in WO2005085226 beschrieben, verabreicht.

Bei einer Ausführungsform der Erfindung wird mindestens eine Verbindung der Formel I in Kombination mit einem CETP-Inhibitor, wie z.B. Torcetrapib oder JTT-705 , verabreicht.

Bei einer Ausführungsform der Erfindung wird mindestens eine Verbindung der Formel I in Kombination mit Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9 beschrieben, verabreicht.

Bei einer Ausführungsform der Erfindung wird mindestens eine Verbindung der Formel I in Kombination mit einem polymeren Gallensäureadsorber, wie z.B. Cholestyramin, Colesevelam, verabreicht.

Bei einer Ausführungsform der Erfindung wird mindestens eine Verbindung der Formel I in Kombination mit einem LDL-Rezeptorinducer (siehe US 6,342,512), wie z.B. HMR1171, HMR1586, oder solchen wie in WO2005097738 beschrieben, verabreicht.

Bei einer Ausführungsform wird mindestens eine Verbindung der Formel I in Kombination mit Omacor® (Omega-3-Fettsäuren; hochkonzentrierte Ethylester der Eicosapentaensäure und der Docosahexaensäure) verabreicht.

Bei einer Ausführungsform der Erfindung wird mindestens eine Verbindung der Formel I in Kombination mit einem ACAT-Inbibitor, wie z.B. Avasimibe, verabreicht.

Bei einer Ausführungsform der Erfindung wird mindestens eine Verbindung der Formel I in Kombination mit einem Antioxidanz, wie z.B. OPC-14117, Probucol, Tocopherol, Ascorbinsäure, β-Caroten oder Selen verabreicht.

Bei einer Ausführungsform der Erfindung wird mindestens eine Verbindung der Formel I in Kombination mit einem Vitamin, wie z. B. Vitamin B6 oder Vitamin B 12 verabreicht.

Bei einer Ausführungsform der Erfindung wird mindestens eine Verbindung der Formel I in Kombination mit einem Lipoprotein-Lipase Modulator, wie z.B. Ibrolipim (NO-1886), verabreicht.

Bei einer Ausführungsform der Erfindung wird mindestens eine Verbindung der Formel I in Kombination mit einem ATP-Citrat-Lyase Inhibitor, wie z.B. SB-204990, verabreicht.

Bei einer Ausführungsform der Erfindung wird mindestens eine Verbindung der Formel I in Kombination mit einem Squalen Synthetase Inhibitor, wie z.B. BMS-188494 oder solchen wie in WO2005077907 beschrieben, verabreicht.

Bei einer Ausführungsform der Erfindung wird mindestens eine Verbindung der Formel I in Kombination mit einem Lipoprotein(a) Antagonist, wie z.B. Gemcabene (CI-1027) verabreicht.

Bei einer Ausführungsform der Erfindung wird mindestens eine Verbindung der Formel I in Kombination mit einem HM74A Rezeptor Agonisten, wie z.B. Nicotinsäure, verabreicht.

Bei einer Ausführungsform der Erfindung wird mindestens eine Verbindung der Formel I in Kombination mit einem Lipase Inhibitor, wie z.B. Orlistat oder Cetilistat (ATL-962), verabreicht.

Bei einer Ausführungsform der Erfindung wird mindestens eine Verbindung der Formel I in Kombination mit Insulin verabreicht.

Bei einer Ausführungsform wird mindestens eine Verbindung der Formel I in Kombination mit einem Sulfonylhamstoff, wie z.B. Tolbutamid, Glibenclamid, Glipizid oder Glimepirid verabreicht.

Bei einer Ausführungsform wird mindestens eine Verbindung der Formel I in Kombination mit einem Biguanid, wie z.B. Metformin, verabreicht.

Bei wieder einer Ausführungsform wird mindestens eine Verbindung der Formel I in Kombination mit einem Meglitinid, wie z.B. Repaglinide oder Nateglinid, verabreicht.

Bei einer Ausführungsform wird mindestens eine Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.

Bei einer Ausführungsform wird mindestens eine Verbindung der Formel I in Kombination mit einem alpha-Glukosidase-Inhibitor, wie z.B. Miglitol oder Acarbose, verabreicht.

Bei einer Ausführungsform wird mindestens eine Verbindung der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform wird mindestens eine Verbindung der Formel I
in Kombination mit mehr als einer der vorstehend genannten Verbindungen, z.B. in Kombination mit einem Sulfonylhamstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer Ausführungsform wird mindestens eine Verbindung der Formel I
in Kombination mit Substanzen, die Einfluss haben auf die hepatische Glukoseproduktion, wie z.B. einem Hemmstoff der Glykogenphosphorylase, wie z.B. PSN-357 oder FR-258900 oder solchen wie in WO2003084922, WO2004007455, WO2005073229-31, WO2005067932 beschrieben, verabreicht.

Bei einer Ausführungsform wird mindestens eine Verbindung der Formel I
in Kombination mit Glukagon-Rezeptor-Antagonisten, wie z.B. A-770077 oder NNC-25-2504 oder solchen wie in WO2004100875, WO2005065680 beschrieben, verabreicht.

Bei einer Ausführungsform wird mindestens eine Verbindung der Formel I
in Kombination mit Aktivatoren der Glukokinase, wie z. B. LY-2121260 (WO2004063179), PSN-105, PSN-110, GKA-50 oder solchen wie sie z. B. in WO2004072031 oder WO2004072066 oder W02005080360 beschrieben sind, verabreicht.

Bei einer Ausführungsform wird mindestens eine Verbindung der Formel I
in Kombination mit einem Inhibitor der Glukoneogenese, wie z. B. FR-225654, verabreicht

Bei einer Ausführungsform wird mindestens eine Verbindung der Formel I
in Kombination mit Inhibitoren der Fructose-1,6-bisphosphatase (FBPase) wie z.B. CS-917, verabreicht.

Bei einer Ausführungsform wird mindestens eine Verbindung der Formel I
in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht

Bei einer Ausführungsform wird mindestens eine Verbindung der Formel I
in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform wird mindestens eine Verbindung der Formel I
in Kombination mit Inhibitoren der Dipeptidylpepridase-IV (DPP-IV), wie z. B. Vildagliptin (LAF-237), Sitagliptin (MK-0431), Saxagliptin ((BMS-477118), GSK-823093, PSN-9301, SYR-322, SYR-619, TA-6666, TS-021, GRC-8200, GW-825964X oder wie sie in WO2003074500, WO2003106456, WO200450658, WO2005058901, WO2005012312, WO2005/012308, PCT/EP2005/007821, PCT/EP2005/008005, PCT/EP2005/008002, PCT/EP2005/008004, PCT/EP2005/008283, DE 10 2005 012874.2 oder DE 10 2005 012873.4 beschrieben sind, verabreicht.

Bei einer Ausführungsform wird mindestens eine Verbindung der Formel I
in Kombination mit Hemmstoffen der 11-beta-Hydroxysteroid-Dehydrogenase-1 (11β-HSD1), wie z. B. BVT-2733 oder solche, wie sie z. B. in WO200190090-94, W0200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004065351, WO2004089367, WO2004089380, WO2004089470-71, WO2004089896, WO2005016877, WO2005097759 beschrieben sind, verabreicht.

Bei einer Ausführungsform wird mindestens eine Verbindung der Formel I
in Kombination mit Inhibitoren der Protein-Tyrosin-Phosphatase-1B (PTP1B), wie sie z. B. in WO200119830-31, WO200117516, WO2004506446, WO2005012295, PCT/EP2005/005311, PCT/EP2005/005321, PCT/EP2005/007151, PCT/EP2005/ oder DE 10 2004 060542.4 beschrieben sind, verabreicht.

Bei einer Ausführungsform wird mindestens eine Verbindung der Formel I
in Kombination mit Modulatoren des natrium-abhängigen Glukosetransporters 1 oder 2 (SGLT1, SGLT2), wie z.B. KGA-2727, T-1095, SGL-0010, AVE 2268 und SAR 7226 oder solchen wie sie z. B. in WO2004007517, WO200452903, W0200452902, PCT/EP200-5/005959, WO2005085237, JP2004359630 oder von A. L. Handlon in Expert Opin. Ther. Patents (2005) 15(11),1531-1540 beschrieben sind, verabreicht.

Bei einer Ausführungsform wird mindestens eine Verbindung der Formel I
in Kombination mit Modulatoren des GPR40 verabreicht.

Bei einer Ausführungsform wird mindestens eine Verbindung der Formel I
in Kombination mit Inhibitoren der hormon-sensitiven Lipase (HSL), wie z. B. in WO2005073199 beschrieben, verabreicht.

Bei einer Ausführungsform wird mindestens eine Verbindung der Formel I
in Kombination mit Hemmstoffen der Acetyl-CoA Carboxylase (ACC) wie z. B. solchen wie in WO199946262, WO200372197, W02003072197, WO2005044814 beschrieben, verabreicht.

Bei einer Ausführungsform wird mindestens eine Verbindung der Formel I
in Kombination mit einem Inhibitor der Phosphoenolpyruvatcarboxykinase (PEPCK), wie z.B. solchen, wie in WO2004074288 beschrieben, verabreicht.

Bei einer Ausführungsform wird mindestens eine Verbindung der Formel 1
in Kombination mit einem Inhibitor der Glykogen Synthase Kinase-3 beta (GSK-3 beta), wie z. B. in US2005222220, WO2005085230, PCT/EP2005/005346, WO2003078403, WO2004022544, WO2003106410, W02005058908, US2005038023, WO2005009997, US2005026984, WO2005000836, WO2004106343, EP1460075, WO2004014910, WO2003076442, WO2005087727, W02004046117 beschrieben.

Bei einer Ausführungsform wird mindestens eine Verbindung der Formel I
in Kombination mit einem Inhibitor der Protein Kinase C beta (PKC beta), wie z. B. Ruboxistaurin, verabreicht.

Bei einer Ausführungsform wird mindestens eine Verbindung der Formel I
in Kombination mit einem Endothelin-A-Rezeptor Antagonisten, wie z. B. Avosentan (SPP-301), verabreicht.

Bei einer Ausführungsform wird mindestens eine Verbindung der Formel I
in Kombination mit Inhibitoren der "I-kappaB kinase" (IKK Inhibitoren), wie sie z. B. in WO2001000610, W02001030774, WO2004022553, WO2005097129 beschrieben sind, verabreicht.

Bei einer Ausführungsform wird mindestens eine Verbindung der Formel I
in Kombination mit Modulatoren des Glucocorticoidrezeptors, wie sie z. B. in WO2005090336 beschrieben sind, verabreicht.

Bei einer weiteren Ausführungsform wird mindestens eine Verbindung der Formel I
in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558);
NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);
Peptid YY 3-36 (PYY3-36) oder analoge Verbindungen wie z. B, CJC-1682 (PYY3-36 konjugiert mit humanem Serum Albumin über Cys34) oder CJC-1643 (Derivat des PYY3-36, welches sich in vivo an Serum Albumin konjugiert) oder solche, wie sie in WO2005080424 beschrieben sind;
Cannabinoid Rezeptor 1 Antagonisten (wie z.B. Rimonabant, SR147778 oder solche wie sie in z. B. EP 0656354, WO 00/15609, WO 02/076949, W02005080345, WO2005080328, WO2005080343, WO2005075450, WO2005080357, W0200170700, WO2003026647-48, WO200302776, WO2003040107, WO2003007887, WO2003027069, US6,509,367, WO200132663, WO2003086288, WO2003087037, WO2004048317, W02004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, W02004096794, WO2005000809, WO2004099157, US20040266845, WO2004110453, WO2004108728, WO2004000817, WO2005000820, US20050009870, WO200500974, W02004111033-34, W0200411038-39, WO2005016286, WO2005007111, WO2005007628, US20050054679, WO2005027837, WO2005028456, WO2005063761-62, WO2005061509, WO2005077897 beschrieben sind);
MC4-Agonisten (z.B. 1-Amino-1,2,3,4-tetrahydro-naphthalin-2-carbonsäure [2-(3a-bezyl-2-methyl-3-oxo-2,3,3a,4,6,7-hexahydro-pymzolo[4,3-c]pyridin-5-yl)-1-(4-chlorophenyl)-2-oxo-ethyl]-amid; (WO 01/91752)) oder LB53280, LB53279, LB53278 oder THIQ, MB243, RY764, CHTR-785, PT-141 oder solche wie sie in WO2005060985, WO2005009950, WO2004087159, WO2004078717, WO2004078716, WO2004024720, US20050124652, WO2005051391, W02004112793, WOUS20050222014, US20050176728, US20050164914, US20050124616, US20050130988, US20040167201, WO2004005324, WO2004037797, WO2005042516, W02005040109, WO2005030797, US20040224901, WO200501921, WO200509184, WO2005000339, EP1460069, WO2005047253, WO2005047251, EP1538159, WO2004072076, WO2004072077 beschrieben sind;
Orexin-Rezeptor Antagonisten (z.B. 1-(2-Methyl-benzoxazol-6-yl)-3-[1,5]naphthyridin-4-yl-hamstoff Hydrochlorid (SB-334867-A) oder solche, wie sie z. B. in WO200196302, WO200185693, WO2004085403, WO2005075458 beschrieben sind);
Histamin H3 Rezeptor Modulatoren (z. B. ABT-834, ABT-239, 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO20006488 WO2005082893, FR2870846
WO2005037810, Celanire, S., et al. Drug Discovery Today 2005, 10, 1613-1627 beschrieben sind);
CRF-Antagonisten (z.B. [2-Methyl-9-(2,4,6-trimethyl-phenyl)-9H-1,3,9-triaza-fluoren-4-yl]-dipropyl-amin (WO 00/66585));
CRF BP-Antagonisten (z.B. Urocortin);
Urocortin-Agonisten;
β3-Agonisten (wie z.B. 1-(4-Chloro-3-methanesulfonylmeyl-phenyl)-2-[2-(2,3-dimethyl-1H-indol-6-yloxy)-ethylamino]-ethanol Hydrochlorid (WO 01/83451));
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2003/15769, WO2005085200, WO2005019240, WO2004011438, WO2004012648, WO2003015769, WO2004072025, WO2005070898, WO2005070925, WO2004039780, WO2003033476, WO2002006245, W02002002744, WO2003004027, FR2868780 beschrieben sind);
CCK-A Agonisten (wie z.B. {2-[4-(4-Chloro-2,5-dimethoxy-phenyl)-5-(2-cyclohexylethyl)-thiazol-2-ylcarbamoyl]-5,7-dimethyl-mdol-1-yl}-essigsäure Trifluoressigsäuresalz (WO 99/15525) oder SR-146131 (WO 0244150) oder SSR-125180);
Serotonin-Wiederaufnahme-Inhibitoren (z.B. Dexfenfluramine);
gemischte Serotonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
5-HT2C Rezeptor Agonisten (wie z.B. APD-356 oder BVT-933 oder solche, wie sie in WO200077010, WO20077001-02, WO2005019180, WO2003064423, WO200242304, WO2005082859 beschrieben sind);
5-HT6 Rezeptor Antagonisten, wie sie z.B. in WO2005038858 beschrieben sind;
Bombesin-Rezeptor Agonisten (BRS-3 Agonisten);
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylamino-ethylcarbamoyl)-3,4-dinhydro-1H-isochinolin-2-carbonsäuretertiärbutylester (WO 01/85695));
Growth Hormone Secretagogue Receptor Antagonisten (Ghrelin Antagonisten) wie z. B. A-778193 oder solchen, wie sie in W02005030734 beschrieben sind;
TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
DA-Agonisten (Bromocriptin, Doprexin);
Lipase/Amylase-Inhibitoren (z.B. WO 00/40569);
Inhibitoren der Diacylglycerol O-Acyltransferasen (DGATs) wie z. B. in US2004/0224997, WO2004094618, WO200058491, WO2005044250, WO2005072740, JP2005206492, WO2005013907 bescbrieben;
Inhibitoren der Fettsäuresynthase (FAS) wie z.B. C75 oder solchen, wie in WO2004005277 beschrieben;
Oxyntomodulin;
Oleoyl-Estron;
oder Agonisten des Schilddrüsenhormonrezeptors (thyroid hormone receptor agonists) wie z. B: KB-2115 oder solche, wie in WO20058279 WO200172692, WO200194293, WO2003084915, WO2004018421, WO2005092316 beschrieben, verabreicht.

Bei einer Ausführungsform der Erfindung ist der weitere Wirkstoff Leptin;
siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff ein Diphenylazetidinonderivat, wie z.B, in US 6,992,067 oder US 7,205,290 beschrieben.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Mazindol oder Phentermin.

Bei einer Ausführungsform wird mindestens eine Verbindung der Formel I
in Kombination mit Ballaststoffen, vorzugsweise unlöslichen Ballaststoffen (siehe z.B. Carob/ Caromax^{®} (Zunft H J; et al., Carob pulp preparation for treatment of hypercholestexolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

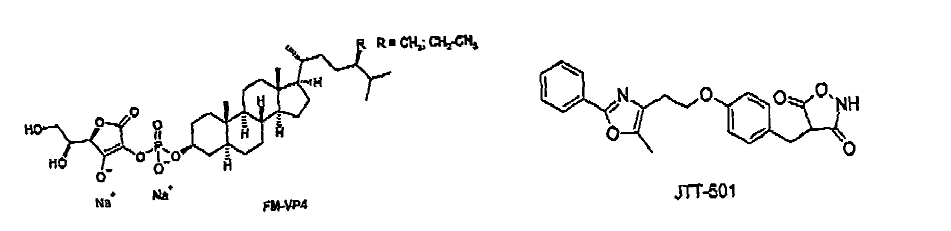

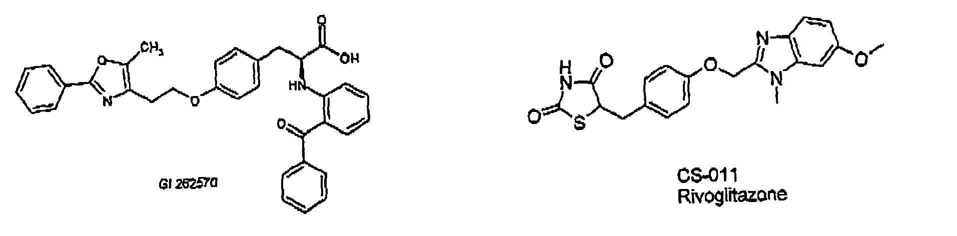

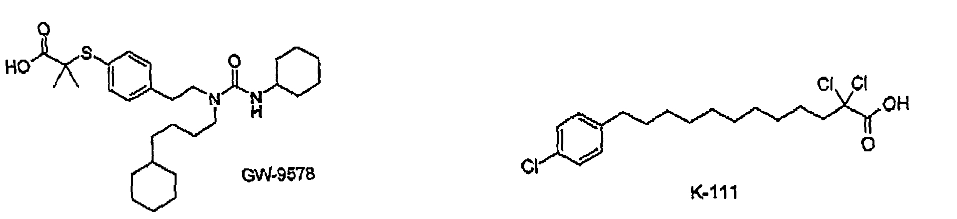

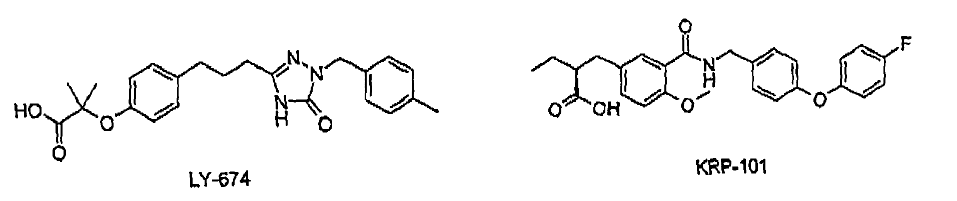

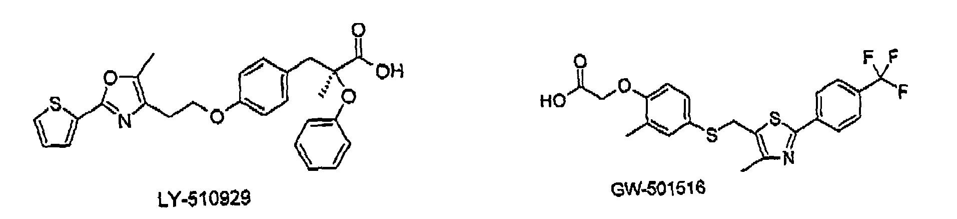

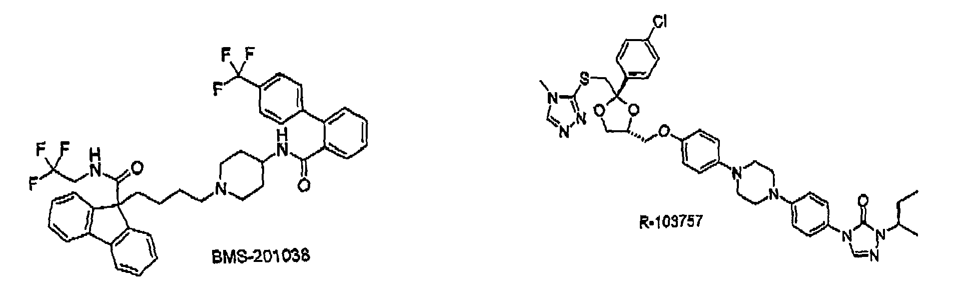

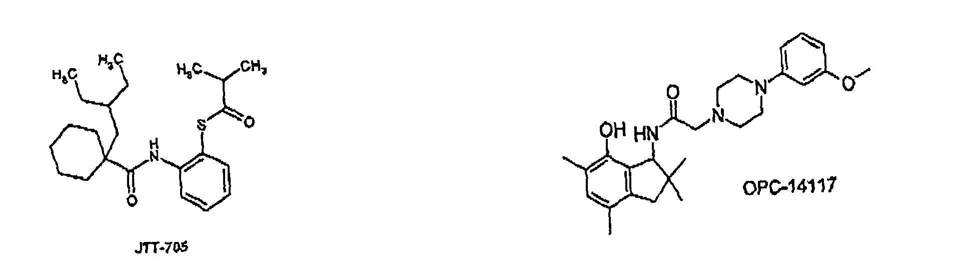

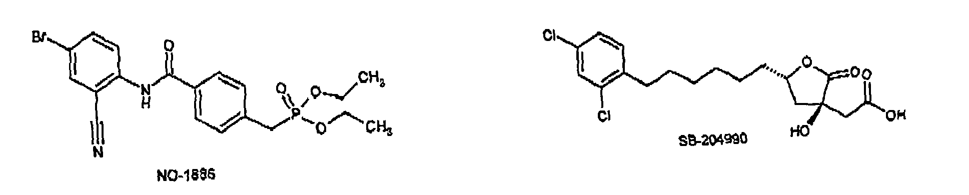

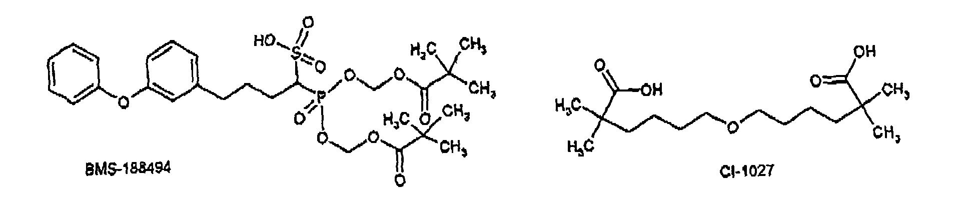

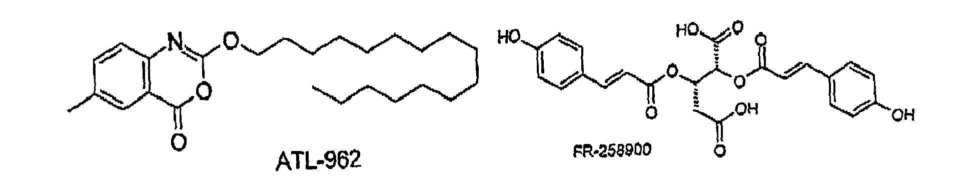

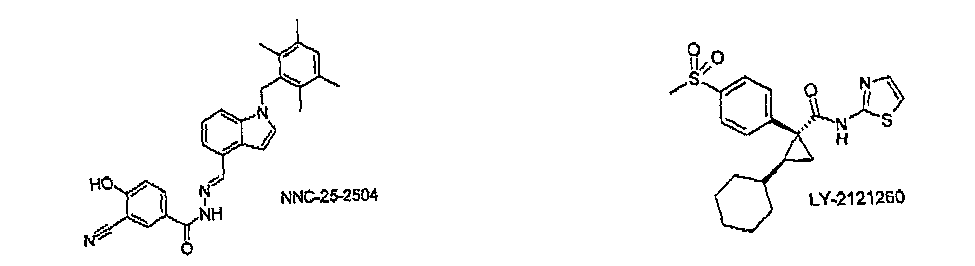

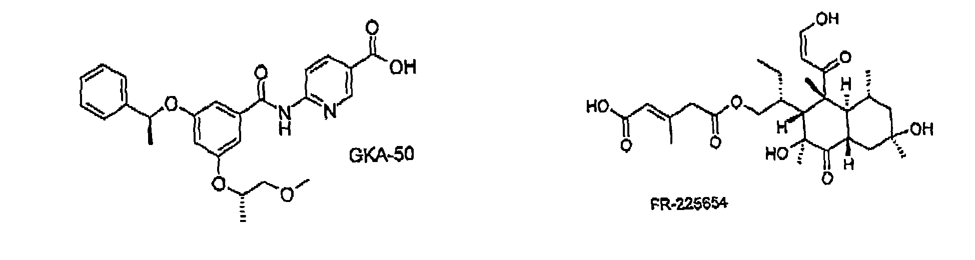

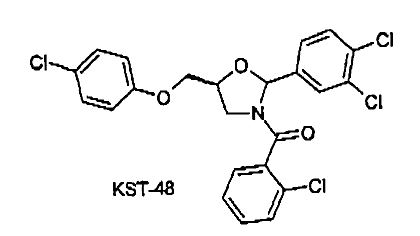

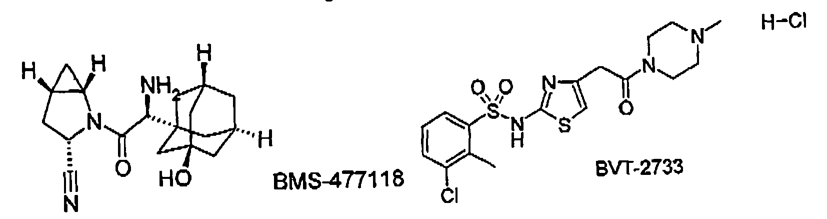

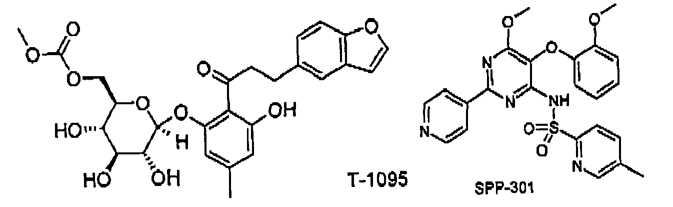

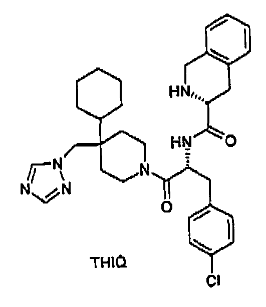

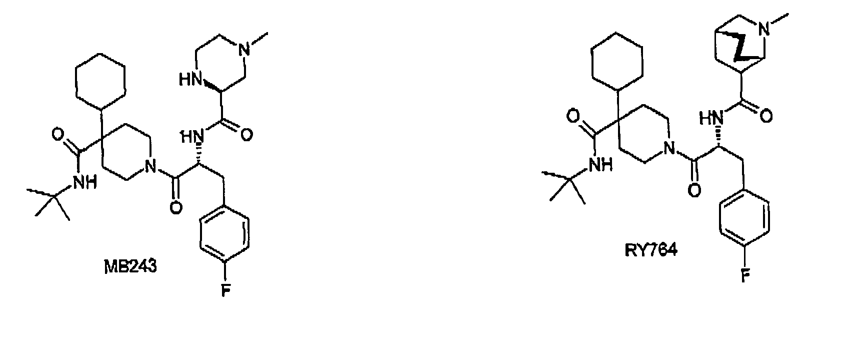

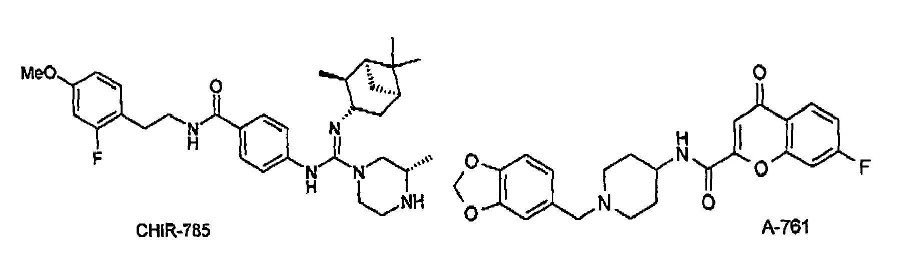

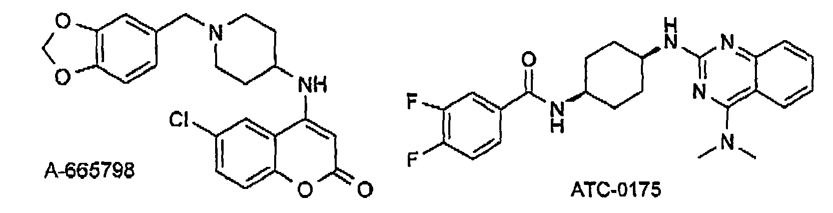

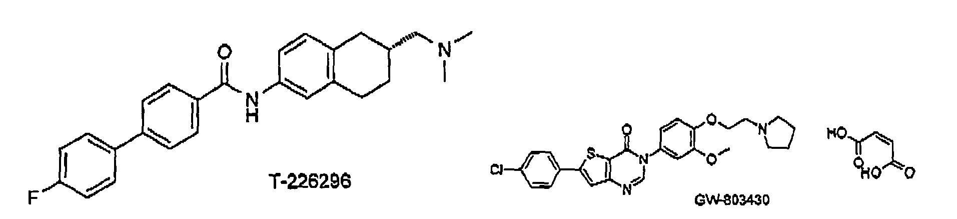

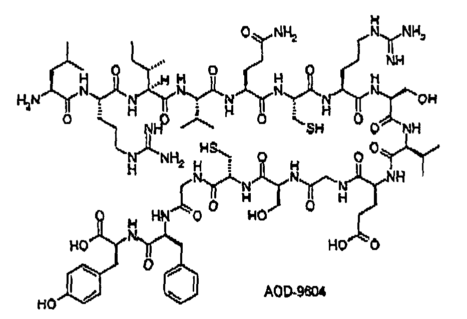

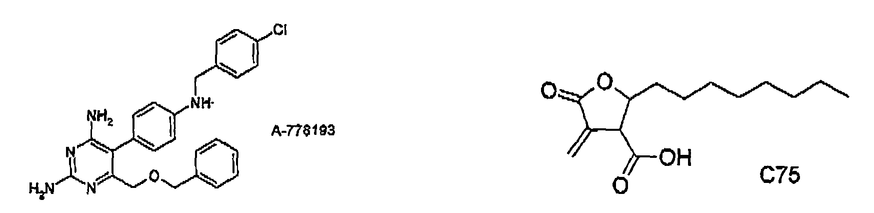

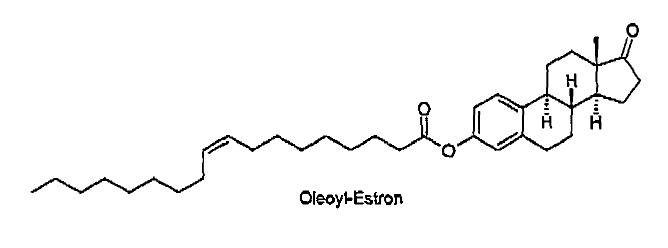

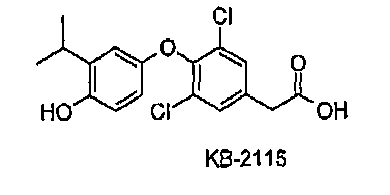

Bei einer Ausfährungsform werden die Verbindungen der Formel I in Kombination mit Medikamenten mit Wirkungen auf das Herz-Kreislauf- und das Blutgefäß-System, verabreicht, wie z.B. ACE-Hemmer (z.B. Ramipril), Medikamente, die auf das Angiotensin-Renin-System wirken, Calcium-Antagonisten, Beta-Blocker etc.

Bei einer Ausführungsform werden die Verbindungen der Formel I in Kombination mit anti-entzündlich wirkenden Medikamenten verabreicht.

Bei einer Ausführungsform werden die Verbindungen der Formel I in Kombination mit Medikamenten, die zur Krebstherapie und Krebsprävention eingesetzt werden, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

### Prüfmodelle

Anhand verschiedener Prüfmodelle kann die Eignung der erfindungsgemäßen Verbindungen als pharmazeutischer Wirkstoffe getestet werden. Im Folgenden werden beispielhaft Beschreibungen solcher Prüfmodelle gegeben.

### Beeinflussung des MCH-Rezeptors in vitro; Bestimmung von funktionellen IC50-Werten von MCH R1-Antagonisten

Die Klonierung der cDNA für den humanen MCH-Rezeptor, Herstellung einer rekombinanten HEK293-Zellinie, welche den humanen MCH-Rezeptor exprimiert, sowie funktionelle Messungen mit der rekombinanten Zellinie erfolgten sinngemäß wie von Audinot et al. (J. Biol. Chem. 2001, 276, 13554-13562) beschrieben. Im Unterschied zur Literaturstelle wurde jedoch für die Konstruktion des Expressionsvektors das Plasmid pEAK8 der Fa. EDGE Biosystems (USA) verwendet. Als Wirt für die Transfektion diente eine transformierte HEK-Zellinie namens "PEAK Stable Cells" (ebenfalls von EDGE Biosystems). Die funktionellen Messungen des zellulären Calciumflusses nach Agonistenzugabe (MCH) in Gegenwart von erfindungsgemäßem Ligand erfolgten mit Hilfe des FLIPR-Crerätes der Fa. Molecular Devices (USA), unter Verwendung von Vorschriften des Geräteherstellers. Die erfindungsgemäßen Verbindungen zeigen eine signifikante Inhibierung (>30%) des durch den Agonisten induzierten Signals bei einer Konzentration von 100 µM, bevorzugt bei 10 µM, besonders bevorzugt bei 1 µM, ganz besonders bevorzugt bei 100 nM und ganz ganz besonders bevorzugt bei 10 nM.
Neben der funktionellen Aktivität kann nach Audinot et al. (Br. J. Pharmacol. 2001, 133, 371-378) auch die Affinität der für den MCH1R bestimmt werden. Bevorzugte erfindungsgemäße Verbindungen zeigen einen IC50-Wert von unter 1 µM, besonders bevorzugt von unter 100 nM, ganz besonders bevorzugt von unter 10 nM und ganz ganz besonders bevorzugt von unter 1 nM.

### Milchaufnahme weiblicher NMRI Mäuse

Die Prüfung der anorektischen Wirkung erfolgt an weiblichen NMRI Mäusen. Nach 24-stündigem Futterentzug wird oder die Prüfsubstanz intraperitoneal oder bevorzugt oral über eine Schlundsonde verabreicht. In Einzelhaltung und bei freiem Zugang zu Trinkwasser wird den Tieren 30 Minuten nach Präparatgabe Kondensmilch angeboten. Der Kondensmilchverbrauch wird halbstündlich 7 Stunden lang bestimmt und das Allgemeinbefinden der Tiere beobachtet. Der gemessene Milchverbrauch wird mit den Vehikel-behandelten Kontrolltieren verglichen.

Das Vehikel hat selbst keinen Einfluss auf die Futteraufnahme. Bevorzugte verträgliche Vehikel für die Applikation sind z. B. Hydroxyethylzellulose (0,5% in Wasser) oder Solutol HS15 (5% in Hydroxyethylzellulose (0,5% in Wasser)).

### Futter- und Wasseraufnahme weiblicher Wistar-Ratten

Alternativ zur Prüfung der anorektischen Wirkung an NMRI Mäusen können auch analog weibliche Wistar-Ratten mit einem Gewicht von ca. 220-250g verwendet werden. Die Tiere werden vor Studienbeginn an die Versuchsumgebung gewöhnt. In einer Ausführungsform haben die Tiere bis zum Beginn des Versuchs freien Zugang zu Futter und Wasser. In einer anderen Ausführungsform wird den Tieren der Zugang zu Futter 24 Stunden vor der Applikation entzogen. Für die Untersuchung der Prüfsubstanz werden die Tiere bei freiem Zugang zu Futter und Wasser einzeln aufgestallt. Futter- und Wasseraufnahme werden mit einem computergestützten System (TSE Drinking & Feeding Monitor) kontinuierlich alle 30 Minuten über einen Zeitraum von 22 Stunden erfasst. Der gemessene Futter- und Wasserverbrauch wird mit den Vehikel-behandelten Kontrollieren verglichen.

### Körpergewichtsentwicklung an diät-induziert adipösen und standard-gefütteren Mäusen

Für diese Untersuchungen werden männliche C57BL6J-Mäuse im Alter von 5 Wochen (Alter des Absetzens) zum einen an eine Standard Haltungsdiät bzw. eine fett- und damit energiereiche Diät gewöhnt. Nach 12 Wochen haben die normalgefütterten, schlanken Mäuse typischerweise ein Körpergewicht von etwa 25 g, die fettgefütterten eines von etwa 35 g erreicht. Die Tiere werden einzeln aufgestallt und individuell die Futter- und Wasseraufnahme bestimmt. Während des Versuches besteht freier Zugang zu Futter und Wasser.
Die Prüfsubstanzen werden in einem Vehikel oral verabreicht und immer im Vergleich zur parallel mitgeführten Vehikel-Kontrolle geprüft. Das Vehikel hat selbst keinen Einfluss auf die Futteraufnahme, üblicherweise handelt es sich um Hydroxyethylzellulose (0,5% in Wasser) oder Solutol HS15 (5% in Hydroxyethylzellulose (0,5% in Wasser)). Zu jeder Gruppe von diät induziert adipösen Mäusen wird eine korrespondierende Gruppe von schlanken Mäusen geführt.

Futter- und Wasseraufnahme werden in der ersten Woche täglich, danach einmal pro Wochen durch Rückwaage des angebotenen Futters bzw. Wassers bestimmt. Das Körpergewicht wird täglich gemessen.

Vor und am Ende der Behandlung werden Blutproben gewonnen, um Serumparameter zu bestimmen, die Hinweise auf Veränderungen des Intermediärstoffwechsels liefern. Weiterhin kann am lebenden Tier der Fettanteil des Körpers mittels einer ImpedanzMessung bestimmt (TOBEC-Methode) werden.

### Mikrokern-Test (in vitro)

Ziel des Mikrokern-Tests (in vitro) ist es, zu prüfen, ob eine Testverbindung das Potential besitzt, die Bildung von Mikrokernen (kleine Membran gebundene DNA-Fragmente) in verschiedenen Zelllinien oder primären Kulturen, mit oder ohne metabolische Aktivierung durch S9-Leberhomogenat, hervorzurufen. Das Testsystem erlaubt die Unterscheidung zwischen dem clastogenen und aneugenen Potential einer Testverbindung durch ein immunochemisches Labelling der Kinetochore oder durch Anfärben der DNA-Fragmente nach dem FISH-(fluorescence in situ hybridization) Verfahren.

Kurzbeschreibung: Die Zellen werden auf einer 96-well Mikrotiterplatte mit der Testverbindung behandelt. Die Behandlungszeit beträgt typischerweise 3 Stunden mit metabolischer Aktivierung beziehungsweise 24 Stunden ohne metabolische Aktivierung. Vierundzwanzig Stunden nach dem Ende der Behandlung werden die Zellen isoliert, fixiert und angefärbt. Die Zytotoxizität der Testverbindung wird beurteilt nach dem relativen Zellwachstum ausgedrückt als Prozentsatz des Wachstums oder unter Berücksichtigung der Verdopplungszeit als Population Doubling im Vergleich zur Negativkontrolle. Die höchste Testkonzentration sollte nicht weniger als 30% überlebende Zellen zeigen, oder sollte die Konzentration sein, bei der ein Niederschlag der Testverbindung beobachtet wird. Doppelbestimmungen sollten bei jeder Testkonzentration durchgeführt werden. Eine genaue ausführliche Versuchsbeschreibung findet sich bei Kirsch-Volders et al. (Mutation Res. 2003, 540, 153-163).

Auswertung: Der strukturelle bzw. numerische Chromosomenschaden wird angegeben als die Erhöhung der Zahl der Zellen mit Mikrokemen in einem Ensemble von 1000 Zellen bei drei analysierbaren Testkonzentrationen. Der Test wird in folgenden Fällen als positiv betrachtet:
a) der Anstieg der Zahl der Zellen mit Mikrokemen ist signifikant im Vergleich zur Negatiwkonttolle (Lösungsmittel bzw. unbehandelt), oder
b) die Zahl der Mikrokerne ist in einem biologisch relevanten Rahmen konzentrationmbhängig erhöht im Vergleich zur Negativkontrolle.

Eine Positivkontrolle muss einen deutlichen statistisch signifikanten Effekt zeigen im Vergleich zur Negativkontrolle.

Bevorzugte Verbindungen der Erfindung sind negativ im Mikrokerntest.

### AMES II-Test

Ziel des AMES II-Tests ist es zu prüfen, ob eine Testverbindung mutagenes Potential besitzt.

Kurzbeschreibung: Auf einer 384-well Mikrotiterplatte wird ein gemischter Bakterienstamm (Mixed strains, 6 verschiedene Salmonella typhimurium Stämme mit jeweils einer Missence-Punktmutation im Histidinoperon) sowie der Salmonella typhimurium Stamm TA98 zum Nachweis von frameshift-Mutationen mit verschiedenen Konzentrationen der Testsubstanz mit oder ohne metabolische Aktivierung durch den Zusatz von S9-Leberhomogenat behandelt (genaue Versuchsbeschreibungen finden sich in der Literatur: P. Gee, D.M. Maron, B.N. Ames; Proc. Natl. Acad. Sci. USA 1994, 91, 11606 bzw. Flückiger-Isler et al.; Mutation Res. 2004, 558, 181 und zit. Lit.).

Mutagene Testverbindungen verursachen Rückmutationen und stellen so die Funktionalität der endogenen Histidinbiosynthese wieder her. Dadurch sind mutierte Bakterien in der Lage sich zu teilen und zu Bakterienkolonien auszuwachsen.

Auswertung: Kommt es zu einem verstärkten Bakterienwachstum bedingt durch Mutationen der Bakterien, so werden Enzyme im Wachstumsmedium verdaut. Dadurch sinkt der pH-Wert im Medium und es erfolgt ein Farbumschlag des zugesetzten Indikators (Bromkresolpurpur) von lila nach gelb. Der Test wird als positiv betrachtet wenn die Anzahl der Welle pro Konzentration, bei denen ein Farbumschlag beobachtet wird, signifikant gegenüber dem Kontrollwert ansteigt.

Bevorzugte Verbindungen der Erfindung sind im AMES II-Test negativ.

### Zvtotoxizitätstests

### a) LDH-Freisetzung

Ziel des Tests auf LDH (Laktat-Dehydrogenase)- Freisetzung ist es zu prüfen, ob eine Verbindung die Integrität der Zellwand schädigt und so den Zelltod verursachen kann.

Kurzbeschreibung: Gemessen wird auf colorimetrischem Weg die LDH-Aktivität, die durch Zellschädigung vom Zytosol in den Zellüberstand gelangt. Die Zellen werden mit der Testverbindung behandelt. Fünfzig Mikroliter des Kulturüberstandes werden abgenommen und mit der Reaktionslösung (LDH-Kit, Roche, Mannheim) nach den Angaben des Herstellers zusammengebracht. LDH katalysiert die Umwandlung von Laktat in Pyruvat. Dabei wird NAD+ zu NADH/H+ reduziert. Letzteres wiederum reduziert unter dem Einfluss der zugesetzten Diaphorase ein ebenfalls zugesetztes gelbes Tetrazoliumsalz zu rotem Formazan.

Auswertung: Die Quantifizierung des Formazans erfolgt durch Messung der. Absorption bei 492 nM (z. B. mit TECAN SPECTRAFluor Plus).

Bevorzugte Verbindungen der Erfindung zeigen keine signifikante Erhöhung der LDH-Aktivität bei Konzentrationen unter 10 µM. Besonders bevorzugte Verbindungen zeigen keine Erhöhung unterhalb einer Konzentration von 50 µM. Noch weiter bevorzugte Verbindungen zeigen keine Erhöhung unterhalb einer Konzentration von 250 µM.

### b) Intrazellulärer ATP-Gehalt

Ziel des Tests ist es, den intrazellulären Gesamt-ATP-Gehalt, der ein Maß für den Energiespiegel und damit die Vitalität einer Zelle ist, zu bestimmen.

Kurzbeschreibung: 100 µL Zellkulturmedium werden in einem Well einer Mikrotiterplatte mit 100 µL des CellTiter-Glo Reagenzes versetzt (folgend den Angaben des Herstellers: Promega Technical Bulletin No. 228, CellTiter-Glo Luminesent Cell Viability Assay). Die Kulturen werden für 2 Minuten bei Raumtemperatur geschüttelt und dann noch 10 Minuten inkubiert bis sich das Lumineszenzsignal stabilisiert hat.

Auswertung: Die Lumineszenz wird über eine Sekunde integrierend aufgenommen (z. B. mit TECAN SPECTRAFluor Plus).

Bevorzugte Verbindungen der Erfindung zeigen keine signifikante Absenkung der ATP-Spiegel bei Konzentrationen unter 10 µM. Besonders bevorzugte Verbindungen zeigen keine Absenkung unterhalb einer Konzentration von 50 µM. Noch weiter bevorzugte Verbindungen zeigen keine Absenkung unterhalb einer Konzentration von 250 µM.

### c) Neutral-Rot-Aufnahme

Ziel des Tests ist es, die Aufnahme von Neutral-Rot (NR) in die Lysosomen / Endosomen und Vakuolen lebender Zellen, die ein quantitatives Maß für die Zahl und Vitalität der Zellen ist, zu messen.

Kurzbeschreibung: Die Zellen werden mit 150 µL einer vorgewärmten Phosphatpufferlösung (PBS) gewaschen und mit 100 µL des NR Mediums für 3 Stunden in einer angefeuchteten Atmosphäre mit 7,5% Kohlendioxid bei 37°C inkubiert. Nach der Inkubation wird das NR-Medium entfernt und die Zellen mit 150 µL PBS gewaschen. Nach dem Entfernen der PBS werden genau 150 µL einer Ethanol / Eisessig-Lösung zugesetzt. Nach 10 Minuten Schütteln ist der Farbstoff aus den Zellen zu einer homogenen Farbstoff-Lösung extrahiert. Eine genaue Beschreibung des Tests findet sich in der Literatur (E. Borenfreund, J.A. Puerner, Toxicol. Lett. 1985, 24(2-3), 119-124).

Auswertung: Die Absorption der Farbstoff-Lösung wird bei 540 nM mittels eines Mikrotiterplattenlesegeräts als Differenz zur Absorption der Ethanol / Eisessig-Lösung bestimmt.

### HERG-Kanal-Blockade

Ziel des Tests ist, den Konzentrationsbereich zu bestimmen, in dem die Testverbindung den kardialen hERG-Kanal blockiert. Eine Blockade des hERG-Kanals, der verantwortlich ist für den Ikr-Strom im menschlichen Herzen, ist mit potentiell tödlichen Arrythmien assoziert.

Die den hERG-Kanal codierende cDNA wurde zur Expression in den pCDNA3-Vektor (Invitrogen) geklont. Eizellen des chinesischen Hamsters (CHO, American Type Culture Collection, Rockville, MD) wurden mittels Lipofectamine (GIBCO/BRL, Grand Island, NY) mit der hERG cDNA trasfiziert und mittels G418 (GIBCO/BRL, Grand Island, NY; 500 µg/mL) selektioniert. CHO-Zellen mit stabiler Expression des hERG-Kanals wurden in einer Atmosphäre von 95% Luft / 5% Kohlendioxid auf einem HAM F-12 Medium kultiviert, das angereichert war mit 10% nativem Rinderserum, 1X Ponicilin/Streptomycin und 500 µg/mL G418.

Die für den Patch-Clamp-Versuch ausgesuchten Zellen werden 18-24 Stunden vor dem Versuch auf einen Plastik-Träger ausgesät. HERG-Kanal-Ströme werden bei Raumtemperatur nach der Ganzzellen-Variante der Patch-Clamp-Technik mit einem Axopatch 200B Verstärker (Axon Instruments, Foster City, CA) aufgezeichnet. Die Elektroden (3-6 Megaohm Widerstand) werden hergestellt aus TW150F Glaskapillaren (World Precision Instruments, Sarasota, FL) and mit der Pipettenlösung (120 mM Kaliumaspartat, 20 mM KCl, 4 mM Na2ATP, 5 mM HEPES, 1 mM MgCl2; auf pH 7,2 gestellt mit KOH) gefüllt. Die hERG-Kanal-Ströme werden ausgelöst durch einen positiven Spannungimpuls (20 mV) gefolgt von einem negativen Impuls (-40 mV) und werden aufgezeichnet für die spätere Analyse. Sobald der hERG-Kanal-Strom der Zelle, die mit der Kontrolllösung (130 mM, 5 mM KCl, 2,8 mM NaOAc, 1 mM MgCl2, 10 mM HEPES; 10 mM Glukose, 1 mM CaCl2; auf pH 7,4 gestellt mit NaOH) gespült wird, stabil ist, wird die Zelle mit der Testverbindung, gelöst in obiger Kontrolllösung (durch Verdünnen einer 10 oder 100 mM DMSO-Lösung der Testverbindung, so dass der DMSO-Anteil nicht mehr als 0,1% beträgt) durchspült, Der Strom wird ständig verfolgt bis keine Änderungen mehr auftreten. Der gleiche Vorgang wird mit steigenden Konzentrationen der Testverbindung wiederholt Für jede Konzentration und für jede Zelle wird die Maximalamplitude des hERG-Stroms in picoAmpere (pA) gemessen. Die Maximalamplitude in pA für jede Konzentration der Testverbindung wird verglichen mit der der reinen Kontrolllösung in derselben Zelle und berechnet als % des Kontrollwertes.

Auswertung: Die Testverbindung wird bei verschiedenen Konzentrationen in 3-5 CHO-Zellen, die den hERG-Kanal exprimieren, getestet. Der IC50-Wert ergibt sich durch Anwendung der nicht-linearen Regression mittels kleinster Fehlerquadrate (GraphPAD Software, San Diego, CA).

### Generelle Selektivität

Um das Risiko unerwünschter Nebenwirkungen zu minimieren, ist es wünschenswert die unselektive Beeinflussung von biologisch wichtigen Funktionseinheiten (z. B. Rezeptoren, Ionenkanäle und Enzyme; für Auflistungen siehe z. B. Whitebread, S. et al.; Drug Discovery Today 2005, 10, 1421-33 und Rolland, C. et al.; J. Med. Chem. 2005, 48, 6563-6574) durch einen pharmazeutischen Wirkstoff möglichst gering zu halten. Generelle Selektivitätstests in einer Vielzahl von in vitro Testsystemen können durch verschiedene spezialisierte Anbieter (z. B. Cerep, Panlabs) durchgeführt werden.

Die erfindungsgemäßen Verbindungen der Formel I weisen als selektive MCH1R-Antagonisten Selektivitätsfaktoren von mindestens 30, bevorzugt von 100, stärker bevorzugt von 300 und noch stärker bevorzugt von 1000 gegenüber der Affinität zu anderen Proteinen auf. Beispiele solcher Proteine sind Serotonin-Rezeptor-Subtypen (z. B. der 5-HT2a Rezeptor), Muscarin-Rezeptor-Subtypen (z. B. der M1-Rezeptor), adrenerge Rezeptorsubtypen (z. B. AR alphala), Natrium- und Calciumkanäle (z. B. der Calciumkanal vom L-Typ).

### Löslichkeiten in wässrigen Systemen

Ausreichende Löslichkeit einer Substanz in wässrigen Lösungsmittelsystemen ist eine wichtige Vorraussetzung für eine (reproduzierbare) pharmakologische Wirkung. Löslichkeiten in wässrigen Systemen können nach verschiedenen Verfahren bestimmt werden. Geeignet sind z. B. Fällungsverfahren aus Lösungen ("kinetische Löslichkeit") und Verfahren, die die Auflösung einer festen Probe bis zur Gleichgewichtseinstellung untersuchen ("thermodynamische Löslichkeit").

### a) Kinetische Löslichkeit

Auf einer 96-well Mikrotiterplatte wird eine DMSO-Lösung der Testverbindung (2,5 mM; 0,5 µL) zu 200 µL einer wässrigen Testlösung (z. B. Phosphate buffered Saline, 10x, 1M, Sigma eingestellt auf 10 mM, pH 7,4) pipettiert und die Trübung bei der so erhaltenen theoretischen Konzentration der Testverbindung von 6,25 µM mittels eines Nephelometers (z. B. Nephelostar Galaxy, BMG Labtech) gemessen. Danach wird die Konzentration der Testverbindung in der wässrigen Testlösung durch Zugabe von weiterer DMSO-Lösung (2,5 mM; 0,5 µL) auf theoretisch 12,5 µM erhöht und die Trübungsmessung erneut durchgeführt. Weitere Zugaben von DMSO-Lösungen (1 µL, 2,5 mM; 0,5 µL, 10 mM; dann 9-mal 1 µL, 10 mM ergebend theoretische Konzentrationen von 25 µM, 50 µM, 100 µM, 150 µM, 200 µM, 250 µM, 300 µM, 350 µM, 400 µM, 450 µM und 500 µM) mit zwischenzeitlicher Trubungsmessung komplettieren den Messprozess.

Auswertung: Die Trübungswerte des Nephelometers werden gegen die theoretische Konzentration der Testverbindung in der wässrigen Testlösung aufgetragen. Sobald bei einer theoretischen Konzentration eine signifikante Trübung detektiert wird (z. B. 5-fach über dem Kontrollwert der wässrigen Testlösung), wird der darunter liegende Konzentrationswert als Löslichkeitsgrenze der Testverbindung in der Testlösung angegeben. Als maximal möglicher Messbereich ergeben sich damit die Werte <6,25 µM, 6,25 - 500 µM und >500 µM.

Bevorzugte erfindungsgemäße Verbindungen zeigen eine kinetische Löslichkeit im Phosphatpuffer (pH 7,4) von mindestens 12,5 µM; bevorzugter von mindestens 50 µM und noch stärker bevorzugt von mindestens 250 µM.

### b) Thermodynamische Löslichkeit

Mittels HPLC-UV-Messung einer Verdünnungsreihe der Testverbindung in DMSO (500 µM, 100 µM, 50 µM, 10 µM und 1 µM) wird die integrierte UV-Absorption in einer Kalibiergeraden linear mit der Konzentration korreliert. Die Testverbindung (500 µg) wird zusammen mit der wässrigen Testlösung (250 µL) in einem geschlossenen Gefäß (Fassungsvolumen: 1,5 mL) für 16 Stunden geschüttelt (Eppendorf Thermoschüttler, 1400 rpm, 25°C, Abdeckung als Liehtschutz). Anschließend wird die Probe bei maximaler Drehzahl zentrifugiert und der Überstand abschließend noch filtriert. Eine Probe des filtrierten Überstandes wird direkt mittels HPLC-UV-Messung (siehe oben) analysiert. Eine weitere Probe wird nach Verdünnen (1 Volumenteil Überstand, 39 Volumenteile Testlösung) analysiert.

Auswertung: Anhand der erstellten Kalibiergeraden wird aus den erhaltenen integrierten UV-Absorptionen der Überstandsproben die Konzentration der Testverbindung im unverdünnten Überstand berechnet und als Löslichkeit der Testverbindung in der jeweiligen wässrigen Testlösung angegeben.

Beispiele für wässrige Testlösungen sind entsalztes Wasser oder wässrige Phosphatpuffer mit verschiedenen pH-Werten (z. B. pH 1,2; pH 4,0; pH 6,8; pH 7,4; pH 9,0), die nach Standardverfahren aus der kommerziellen Lösung (Phosphate buffered saline, 10x, Sigma) hergestellt werden können durch Verdünnen bzw. Einstellen mit Phosphorsäure oder Natronlauge.

Bevorzugte erfindungsgemäße Verbindungen zeigen eine Löslichkeit im Phosphatpuffer (pH 7,4) von mindestens 12,5 µM; bevorzugter von mindestens 50 µM und noch stärker bevorzugt von mindestens 250 µM.

### Permeabilität

Der Test auf Permeabilität wird in CACO-2/TC7 Zellen durchgeführt, die für 21 Tage auf Becton Dickinson-Filtern (24-well, nicht beschichtet) kultiviert wurden (DMEM / Glutamax I / Gibco mit hohem Glukoseanteil, HEPES 25 mM, 1% NEAA, 10% FBS, 40 µg/mL Gentamycin; 37°C Umgebungstemperatur; 95% Luftfeuchtigkeit und 10% CO2-Gehalt). Die Permeabilität wird bei einer Konzentration der Testverbindung von 20 µM (1% DMSO in HBSS) mit einem pH-Gradienten (apical: pH 6,5 und 0,5% BSA; basolateral: pH 7.4 und 5% BSA) geprüft. Die Analyse erfolgt mittels LCMS/MS. Weitere Beschreibungen des Testsystems und Referenzen zur experimentellen Durchführung finden sich bei Balimane, P.V.; Drug Discovery Today 2005, 10(5), 335-343.

### Inhibition von CYP-Enzymen

Die Inhibition von CYP-Enzymen wird an rekombinanten Enzymen (erhalten von Becton Dickinson) und fluoreszierenden Substraten (BD/Gentest) nach den Empfehlungen des Herstellers bestimmt (siehe Website http://www.bdbiosciences.com). Weitere Beschreibungen des Testsystems und Referenzen zur experimentellen Durchführung finden sich bei Zlokarnik, G.; Drug Discovery Today 2005, 10(21), 1443-1450.

### Metabolische Stabilität

Die metabolische Stabilität wird bestimmt durch Inkubation der Testverbindung (5 µM) bei 37°C mit microsomalen Leberfraktionen (1 mg/mL Protein mit 0,1 % w/v BSA; 1 mM NADPH, 0,5% DMSO). Die Analyse bei 0 und 20 Minuten Inkubationszeit erfolgt mittels LCMS/MS. Weitere Beschreibungen des Testsystems und Referenzen zur experimentellen Durchführung finden sich bei Plant, N.; Drug Discovery Today 2004, 9(7), 328-336 und Lau, Y.Y. et al.; Pharmaceutical Res. 2002, 19(11), 1606-1610.

### Beispiele

Die nachfolgend aufgeführten Beispiele und Herstellungsmethoden dienen zur Erläuterung der Erfindung, ohne diese jedoch einzuschränken.

Die erfindungsgemäßen Verbindungen der Formel I können mit Hilfe von im Prinzip bekannten Reaktionen hergestellt werden. Zum Beispiel kann eine Aminosäure der Struktur Z1 zunächst selektiv geschützt werden (z. B. nach Methode D mit Boc2O). Die folgende Reaktion mit einem Amin (HNR1R2) kann vorteilhaft unter Verwendung eines allgemein bekannten Kupplungsreagenzes (z. B. nach Methode A mit HATU) durchgeführt werden. Entferung der Schutzgruppe (z. B. nach Methode C mit Salzsäure) und anschliessende Reduktion (z. B. nach Methode B mit Lithiumaluminiumhydrid) ergibt ein Amin der Struktur Z2 (wobei R8 = H). Entfernt man eine Carbamatschutzgruppe vor der Reduktion nicht, wird Z2 mit R8 = Methyl erhalten. In einem letzten Schritt können die erfindungsgemäßen Verbindungen der Formel Ia durch Reaktion des Amins Z2 mit einer Säure der Struktur Z3 (z. B. nach Methode A) erhalten werden (Schema 1).

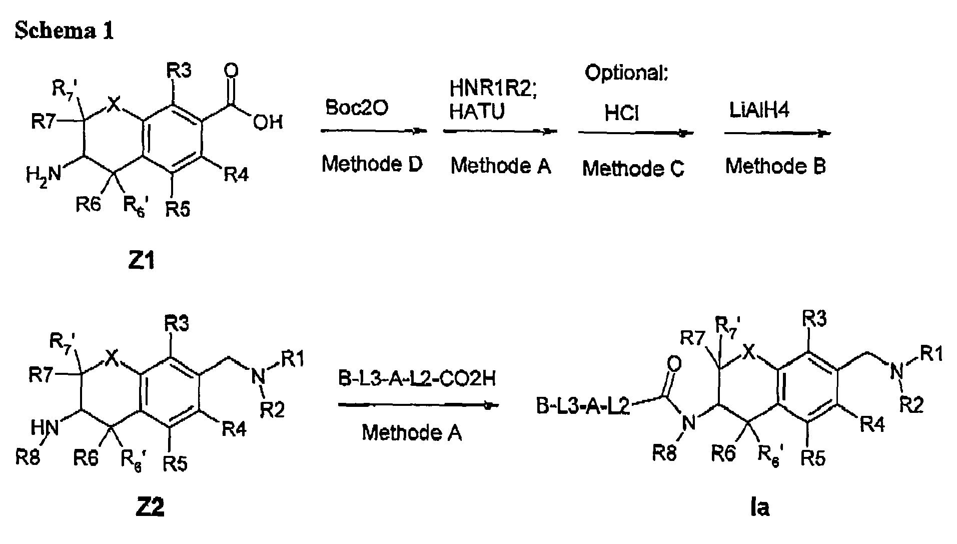

Alternativ können Verbindungen der Formel I aus den Ketonen Z3 erhalten werden, welche kommerziell erhätlich sind, oder nach bekannten Methoden (siehe z. B. Synthesis 2004, 121; J. Org. Chem. 1995, 60, 4324) hergestellt werden können. Säurekatalysierte Kondensation der Ketone Z3 mit Amiden (B-L3-A-L2-CONH2) und anschliessende (optional asymmetrische) katalytische Hydrierung der erhaltenen Enamide unter bekannten Bedingungen (siehe z. B. Adv. Synth. Catal. 2003, 345, 230; Tetrahedron: Asymmetry 1999, 10, 3467; J. Org. Chem. 1995, 60, 4324) ergibt die Arylbromide Z4. Diese können nach Literaturvorschriften in die Arylcarbonylverbindungen Z5 umgewandelt werden (siehe z. B. J. Am. Chem. Soc. 2000, 122, 6935; J. Med. Chem. 2005, 48, 1948; Angew. Chem. Int. Ed. 2006, 45, 154). Abschliessende reduktive Aminierung führt zu den Verbindungen Ib (Schema 2).

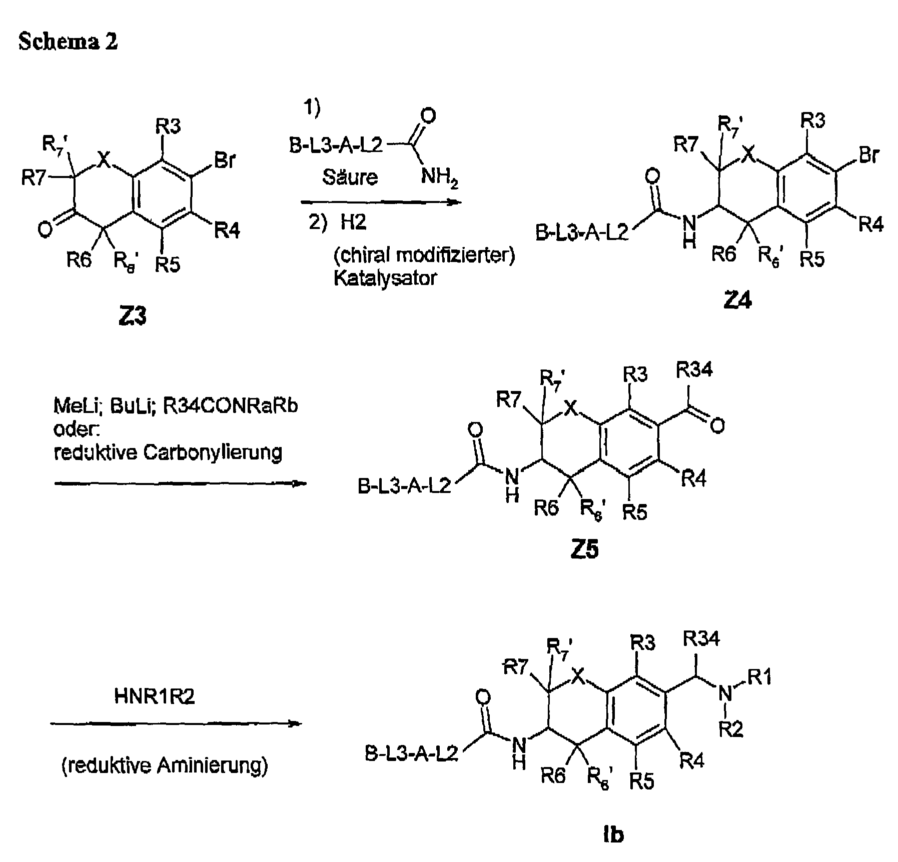

Ein weiteres Herstellungsverfahren für die Verbindungen der Formel I besteht darin Dichloride des Typs Z6 oder Isochromenone Z7 mit Aminen Z8 nach im Prinzip bekannten Verfahren umzusetzen (Schema 3). Die benötigten Dichloride Z6 können aus ortho-Methylbenzoesäuren durch doppelte Metallierung z. B. mit Lithium-diisopropylamid (LDA), Abfangen des Dianions mit Formaldehyd (z. B. in Form von Paraformaldehyd) und abschliessende Dichlorierung mit SOCl₂ gewonnen werden. Die Amine Z8 können z. B. nach Schema 1 (Z2 mit R8 = H) oder nach Schema 2 durch Hydrolyse der Strukturen Ib erhalten werden.

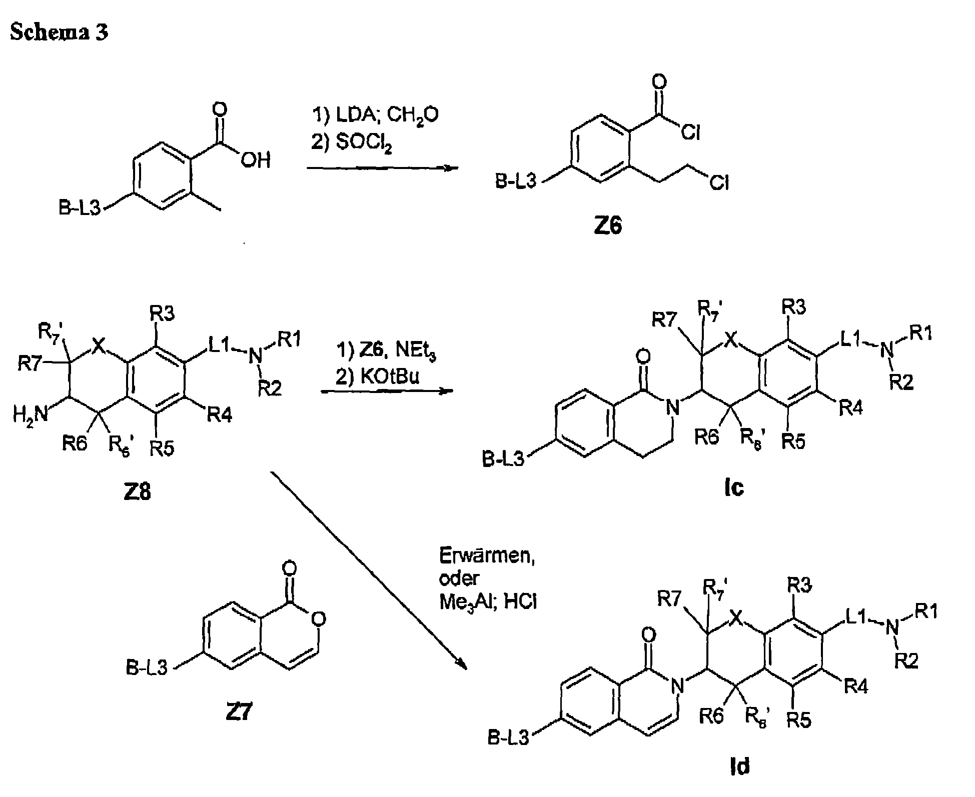

Beschreibungen der verwendeten allgemeinen Methoden finden sich exemplarisch an folgenden Stellen beschrieben:
Methode A, B, C, D, E, F, G im Beispiel 1;

### Allgemeine Erläuterungen

### a) Zeichenweise der Strukturformeln

In den Strukturformeln der gegebenen Beispiele sind zur Übersichtlichkeit nur Nicht-Wasserstoffatome dargestellt.

### b) Salzformen

Viele der erfindungsgemäßen Verbindungen sind Basen und können mit entsprechend starken Säuren Salze bilden. Insbesondere können die Verbindungen nach HPLCchromatograpischer Reinigung unter Verwendung eines Trifluoressigsäure enthaltenden Laufmittels als Hydrotrifluoracetate vorliegen. Diese können durch einfaches Behandeln einer Lösung der Salze z. B. mit Natriumcarbonatlösung in die gezeigten freien Basen überführt werden.

### c) Einheiten der Charakterisierungsdaten

Die Einheit der angegebenen Molekulargewichte ist "g/mol". Beobachtete Peaks im Massenspektrum sind angegeben als ganzzahliger Quotient der molaren Molekülionmasse und der Ladung des Molekülions (m/z).

### Beispiel 1

### N-((S)-6-Pyrrolidin-1-ylmethyl-1,2,3,4-tetrahydro-naphthalen-2-yl)-4-[(S)-1-(tetrahydrofuran-2-yl)methoxy]-benzamide

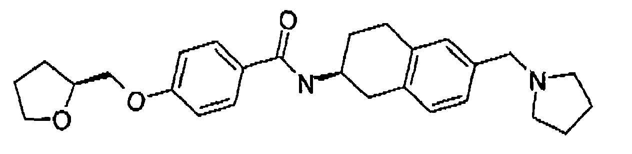

### Methode A

Zu einer Lösung von 4-[(S)-1-(TETRAHYDRO-FURAN-2-YL)METHOXY]-BENZOIC ACID (3,38 g) in NMP (30 mL) wurde HATU (O-(7-AZABENZOTRIAZOL-I-YL)-N,N,N',N'-TETRAMETHYLURONIUM-HEXAFLUOROPHOSPHATE; 5,78 g)) und dann Triethylamin (2,12 mL) gegeben. Es wurde eine Lösung von (S)-6-Pyrrolidin-1-ylmethyl-1,2,3,4-tetrahydro-naphthalen-2-ylamine (3,5 g) in NMP (20 mL) zugetropft. Nach 12 Stunden wurde die Reaktionsmischung mit Ethylacetat verdünnt, mit gestättigter Natriumcarbonatlösung gewaschen und eingengt. Der Rückstand wurde durch Chromatographie an Kieselgel (Laufmittel DCM / MeOH 10:1) gereinigt. Man erhielt so das Produkt mit dem Molekulargewicht 434,58 (C27H34N2O3); MS (ESI): 435 (M+H+).

### (S)-6-Pyrrolidin-1-ylmethyl-1,2,3,4-tetrahydro-naphthalen-2-ylamine

### Methode B

Eine Lösung von ((S)-6-Amino-5,6,7,8-tetrahydro-naphthalen-2-yl)-pyrrolidin-yl-methanone (0,49 g) in THF (5 mL) wurde zu einer Suspension von Lithiumaluminiumhydrid (0,60 g) in THF (10 mL) getropft. Die Mischung wurde für eine Stunde bei RT gerührt. Es wurde Wasser (0,6 mL), gefolgt von Natronlauge (16%ig; 2 mL) und wieder Wasser (2 mL) vorsichtig zugetropft. Der entstandene Niederschlag wurde abfiltriert und das Filrat eingeengt. Der Rückstand wurde in Salzsäure (1N) aufgenommen und die Lösung mit Diethylether gewaschen. Die wässrige Phase wurde mit konzentrierter Natronlauge basisch gestellt und mit DCM dreimal extrahiert. Die vereinigten organischen Phasen wurden über Magnesiumsulfat getrocknet und eingeengt. Man erhielt so das Produkt mit dem Molekulargewicht 230,36 (C15H22N2); MS (ESI): 231 (M+H+).

### ((S)-6-Amino-5,6,7,8-tetrahydro-naphthalen-2-yl)-pyrrolidin-1-yl-methanone

### Methode C

Zu einer Lösung von [(S)-6-(Pyzzalidine-1-carbonyl)-1,2,3,4-tetrahydro-naphthalen-2-yl]-carbamic acid tert-butyl ester (0,70 g) in MeOH (5 mL) wurde konzentrierte Salzsäure (5 mL) gegeben. Nach einer Stunde wurde die Mischung mit konzentrierter Natronlauge basisch gestellt und mit DCM dreimal extrahiert. Die vereinigten organischen Phasen wurden über Magnesiumsulfat getrocknet und eingeengt. Man erhielt so das Produkt mit dem Molekulargewicht 244,34 (C15H20N2O); MS (ESI): 245 (M+H+).

### [(S)-6-(Pyrrolidine-1-carbonyl)-1,2,3,4-tetrahydro-naphthalen-2-yl]-carbamic acid tert-butyl ester

Nach Methode A wurde (S)-6-tert-Butoxycarbonylamino-5,6,7,8-tetrahydro-naphthalene-2-carboxylic acid mit Pyrrolidin umgesetzt. Man erhielt so das Produkt mit dem Molekulargewicht 344,46 (C20H28N203); MS (ESI): 345 (M+H+).

### (S)-6-tert-Butoxycarbonylamino-5,6,7,8-tetrahydro-naphthalene-2-carboxylic acid

### Methode D

Zu einer Mischung von (S)-6-Amino-5,6,7,8-tetrahydro-naphthalene-2-carboxylic acid (1,0 g), Natronlauge (32%ig; 1,1 g) und MeOH (20 mL) wurde DI-TERT-BUTYL DICARBONATE (1,92 g) gegeben. Die Mischung wurde bei 50°C für 6 Stunden gerührt und dann Wasser (150 mL) hinzugefügt. Nach der Extraktion mit Diethylether wurde die wässrige Phase leicht angesäuert und dreimal mit DCM extrahiert. Die vereinigten organischen Phasen wurden über Magnesiumsulfat getrocknet und eingeengt, Man erhielt so das Produkt mit dem Molekulargewicht 291,35 (C16H21NO4); MS (ESI): 292 (M+H+).

### 4-[(S)-1-(Tetrahydro-furan-2-yl)methoxy]-benzoic acid

### Methode E

Eine Mischung aus 4-[(S)-1-(Tetrahydro-furan-2-yl)methoxy]-benzoic acid methyl ester (9,8 g), Natronlauge (2N; 80 mL) und MeOH (300 mL) wurde für 12 Stunden gerührt. Organische flüchtige Anteile wurden am Rotationsverdampfer entfernt. Die verbleibende wässrige Phase wurde mit MTBE extrahiert und dann mit konzentrierter Salzsäure sauer gestellt. Der entstandene Niederschlag wurde abfiltriert und getrocknet. Man erhielt so das Produkt mit dem Molekulargewicht 222,24 (C12H14O4); MS (ESI): 223 (M+H+).

### 4-[(S)-1-(Tetrahydro-furan-2-yl)methoxy]-benzoic acid methyl ester

### Methode F

Eine Mischung aus Methanesulfonic acid (S)-1-(tetrahydro-furan-2-yl)methyl ester (7,6 g), 4-Hydroxy-benzoic acid methyl ester (6,4 g), Cäsiumcarbonat (20 g) und NMP (100 mL) wurde für 12 Stunden auf 75°C erwärmt. Die abgekühlte Reaktionsmischung wurde mit Wasser versetzt und mit Ethylacetat extrahiert. Die organische Phase wurde dreimal mit Wasser gewaschen, über Magnesiumsulfat getrocknet und eingeengt. Man erhielt so das Produkt mit dem Molekulargewicht 236,27 (C13H16O4); MS (ESI): 237 (M+H+).

### Methanesulfonic acid (S)-1-(tetrahydro-furan-2-yl)methyl ester

### Methode G

Zu einer Lösung von (S)-1-(Tefrahydro-furan-2-yl)-methanol (7,95 g) in Pyridin (35 mL) wurde bei -15 °C Methansulfonsäurechlorid (7,47 g) zugegeben und die Reaktion wurde für 5 Stunden bei 0 °C gerührt. Nach der Zugabe von Wasser wurde das Gemisch mit Ethylacetat extrahiert. Die organischen Phasen wurden über Magnesiumsulfat getrocknet und eingeengt. Man erhielt so das Produkt mit dem Molekulargewicht 180,22 (C6H12O4S); MS (ESI): 181 (M+H+).

### Beispiel 2

### N-Methyl-N-((S)-6-pyrrolidin-1-ylmethyl-1,2,3,4-tetrahydro-naphthalen-2-yl)-4-[(S)-1-(tetrahydro-furan-2-yl)methoxy]-benzamide

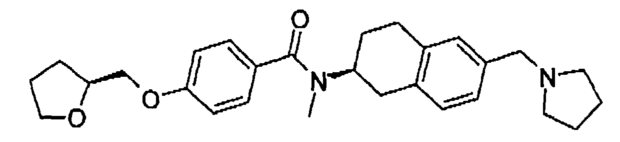

Nach Methode A wurde 4-[(S)-1-(TETRAHYDRO-FURAN-2-YL)METHOXY]-BENZOIC ACID mit Methyl-((S)-6-pyrrolidin-1-ylmethyl-1,3,4-tetrahydro-naphthalen-2-yl)-amine umgesetzt. Man erhielt so das Produkt mit dem Molekulargewicht 448,61 (C28H36N2O3); MS (ESI): 449 (M+H+).

Methyl-((S)-6-pyrrolidin-1-ylmethyl-1,2,3,4-tetrahydro-naphthalen-2-yl)-amine Nach Methode B wurde [(S)-6-(Pyrrolidine-1-carbonyl)-1,2,3,4-tetralaydro-naphthalen-2-yl]-carbamic acid tert-butyl ester mit Lithiumaluminiumhydrid (10 Äquiv., 60°C, 2 Stunden) umgesetzt. Man erhielt so das Produkt mit dem Molekulargewicht 244,38 (C16H24N2); MS (ESI): 245 (M+H+).

Die Tabelle 1 enthält Illustrationsbeispiele, die mögliche aber nicht begrenzende Ausführungsformen der Erfindung darstellen.

**Tabelle 1.**

| Bsp. No. | Struktur | Bsp. No. | Struktur |
|---|---|---|---|
| I1 | 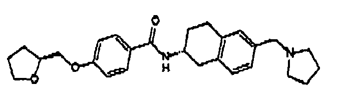 | I13 | 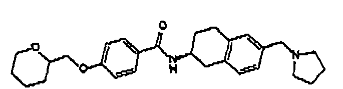 |
| I2 | 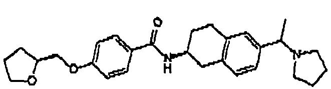 | I14 | 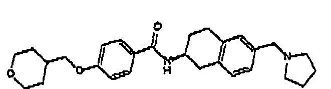 |
| 13 | 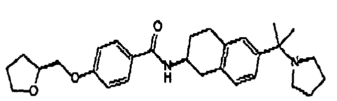 | I15 | 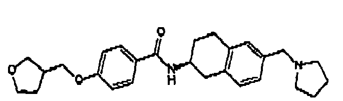 |
| I4 | 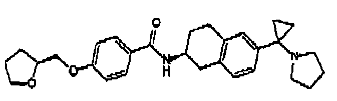 | I16 | 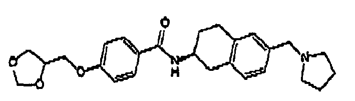 |
| 15 | 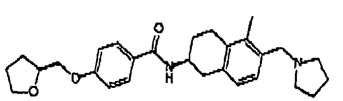 | I17 | 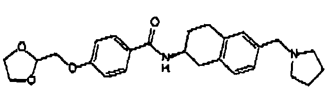 |
| I6 | 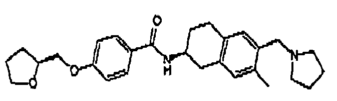 | I18 | 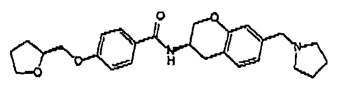 |
| I7 | 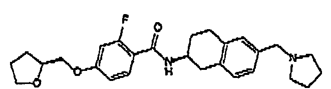 | I19 | 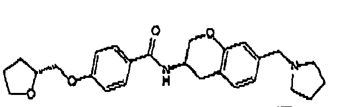 |
| I8 | 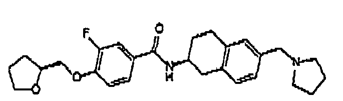 | I20 | 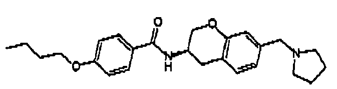 |
| 19 | 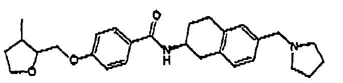 | I21 | 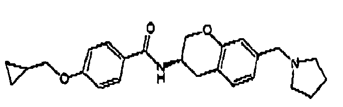 |
| I10 | 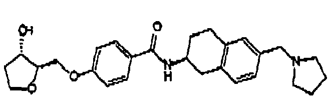 | I22 | 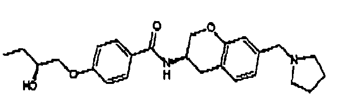 |
| I11 | 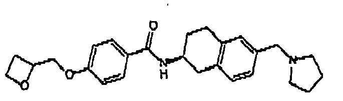 | I23 | 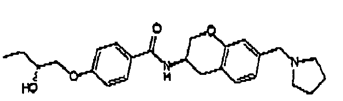 |
| I12 | 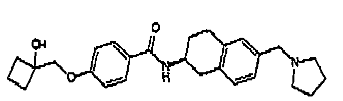 | I24 | 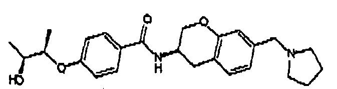 |
| I25 | 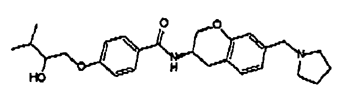 | I37 | 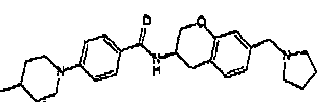 |
| I26 | 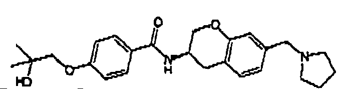 | I38 | 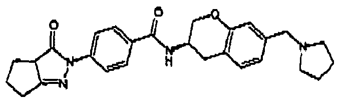 |
| I27 | 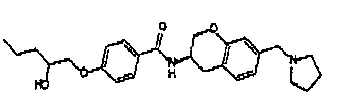 | I39 | 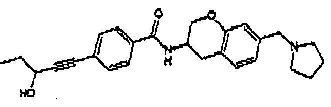 |
| I28 | 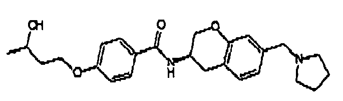 | I40 | 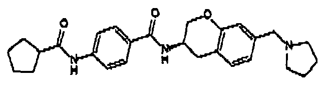 |
| I29 | 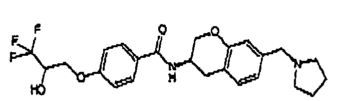 | I41 | 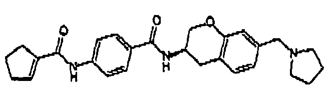 |
| I30 | 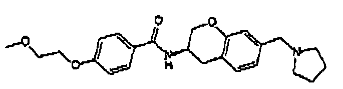 | I42 | 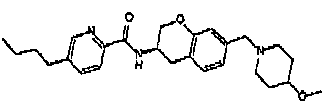 |
| I31 | 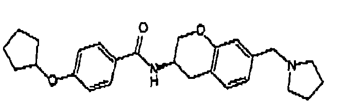 | I43 | 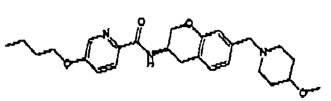 |
| I32 | 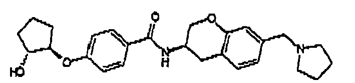 | I44 | 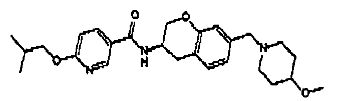 |
| I33 | 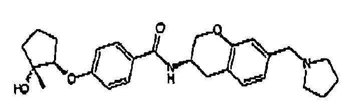 | I45 | 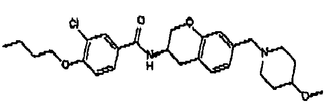 |
| I34 | 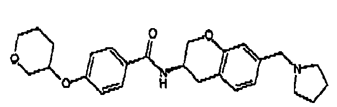 | I46 | 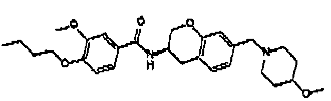 |
| I35 | 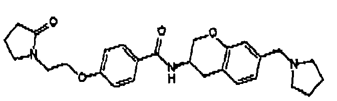 | I47 | 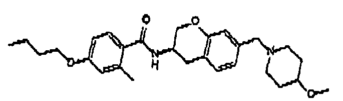 |
| I36 | 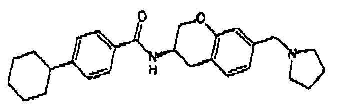 | I48 | 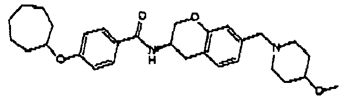 |
| I49 | 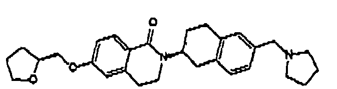 | I61 | 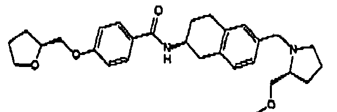 |
| I50 | 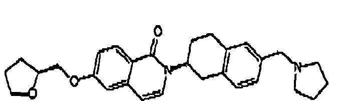 | I62 | 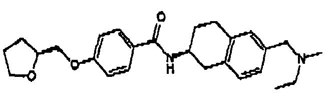 |
| I51 | 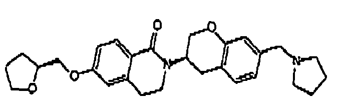 | I63 | 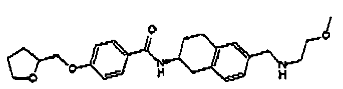 |
| I52 | 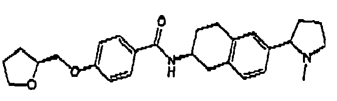 | I64 | 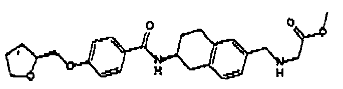 |
| I53 | 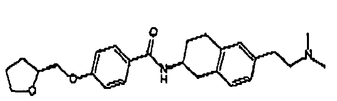 | I65 | 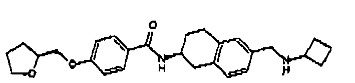 |
| I54 | 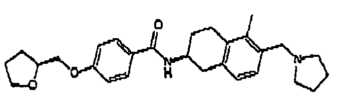 | I66 | 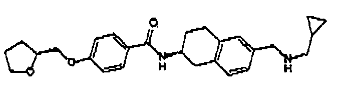 |
| I55 | 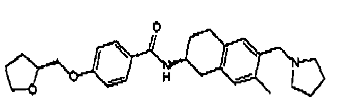 | I67 | 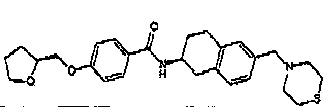 |
| I56 | 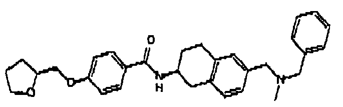 | I68 | 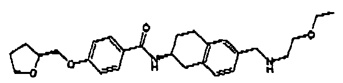 |
| I57 | 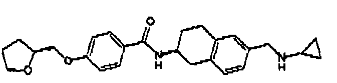 | I69 | 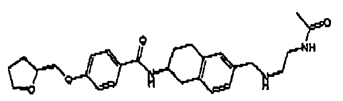 |
| I58 | 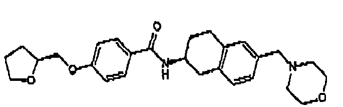 | I70 | 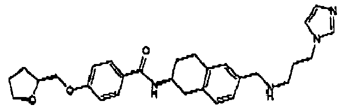 |
| I59 | 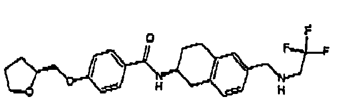 | I71 | 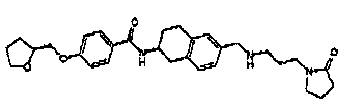 |
| I60 | 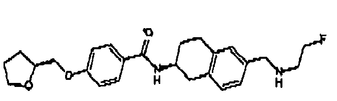 | I72 | 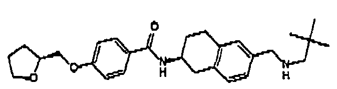 |
| I73 | 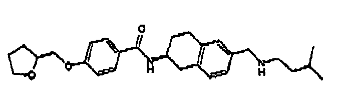 | I85 | 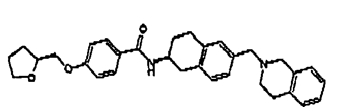 |
| I74 | 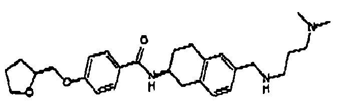 | I86 | 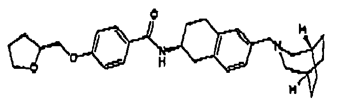 |
| I75 | 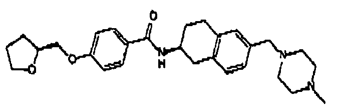 | I87 | 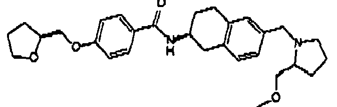 |
| I76 | 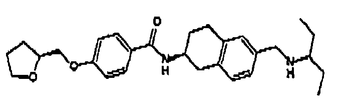 | I88 | 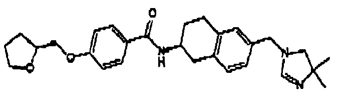 |
| I77 | 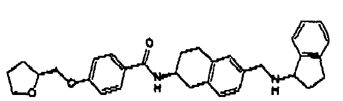 | I89 | 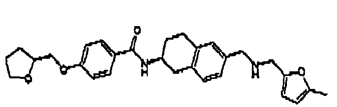 |
| I78 | 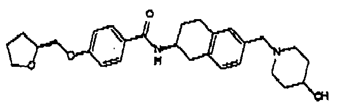 | I90 | 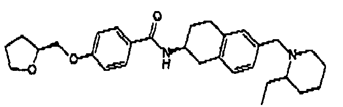 |
| I79 | 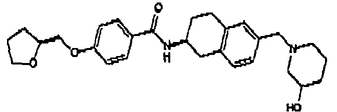 | I91 | 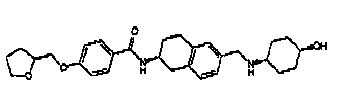 |
| I80 | 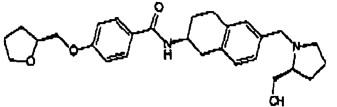 | I92 | 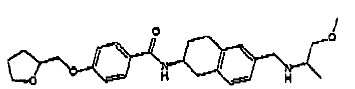 |
| I81 | 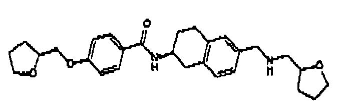 | I93 | 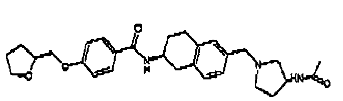 |
| I82 | 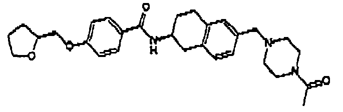 | I94 | 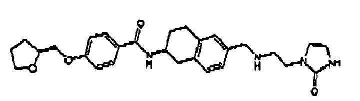 |
| I83 | 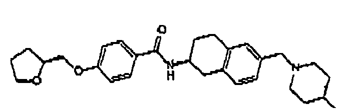 | I95 | 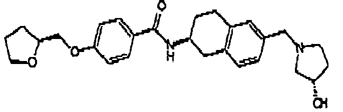 |
| I84 | 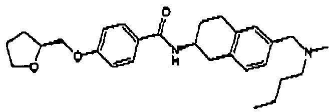 | I96 | 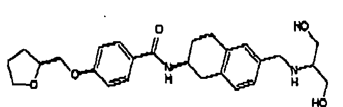 |
| I97 | 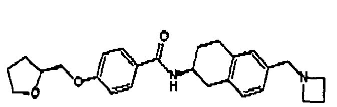 | I109 | 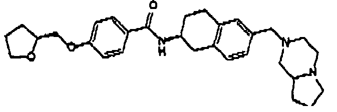 |
| I98 | 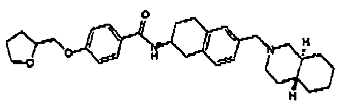 | I110 | 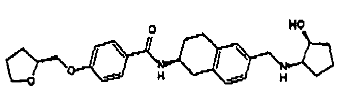 |
| I99 | 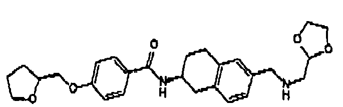 | I111 | 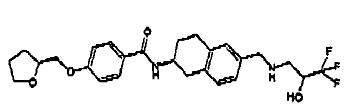 |
| I100 | 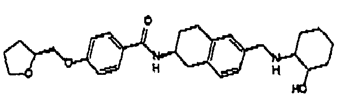 | I112 | 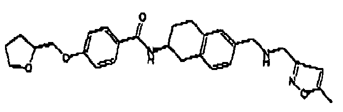 |
| I101 | 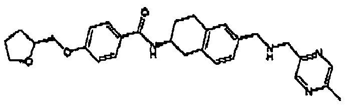 | I113 | 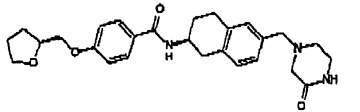 |
| I102 | 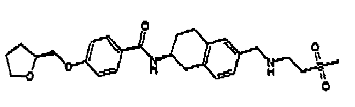 | I114 | 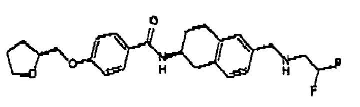 |
| I103 | 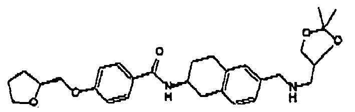 | I115 | 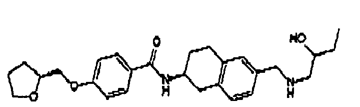 |
| I104 | 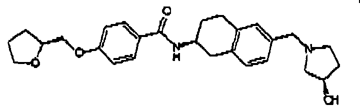 | I116 | 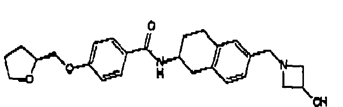 |
| I105 | 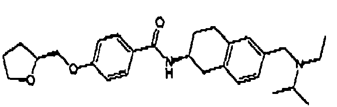 | I117 | 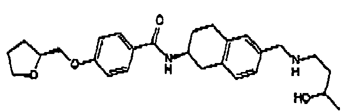 |
| I106 | 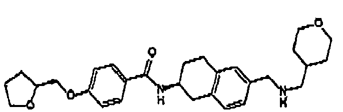 | I118 | 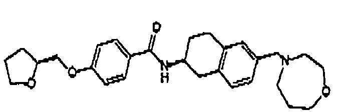 |
| I107 | 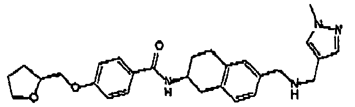 | I119 | 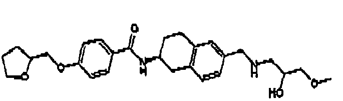 |
| I108 | 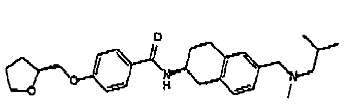 | I120 | 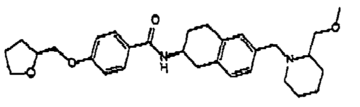 |
| I121 | 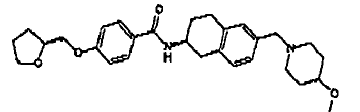 | I133 | 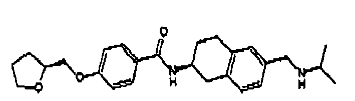 |
| I122 | 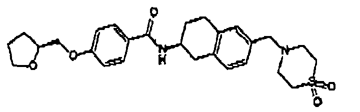 | I134 | 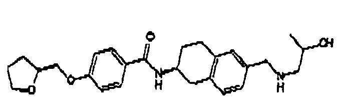 |
| I123 | 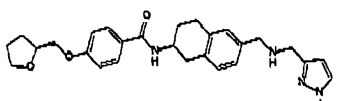 | I135 | 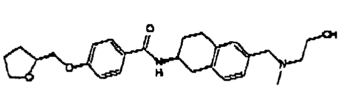 |
| I124 | 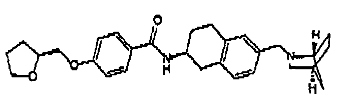 | I136 | 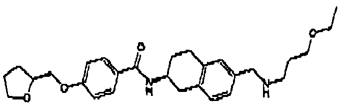 |
| I125 | 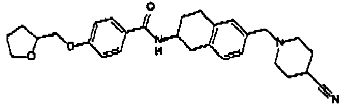 | I137 | 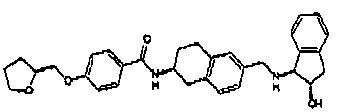 |
| I126 | 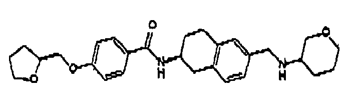 | I138 | 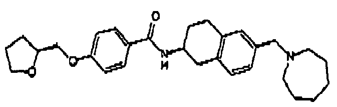 |
| I127 | 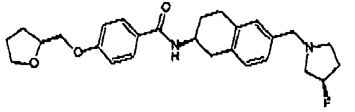 | I139 | 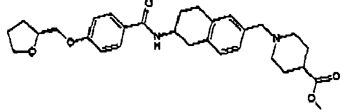 |
| I128 | 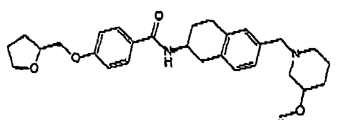 | I140 | 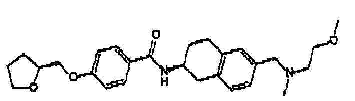 |
| I129 | 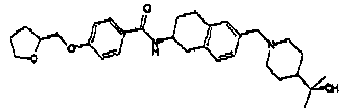 | 1141 | 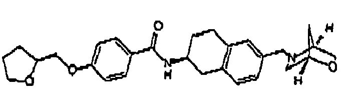 |
| I130 | 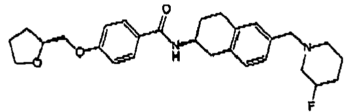 | I142 | 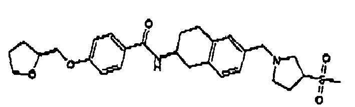 |
| I131 | 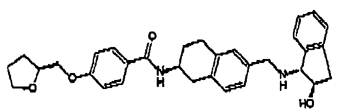 | I143 | 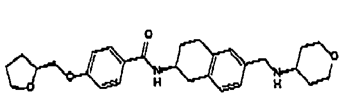 |
| I132 | 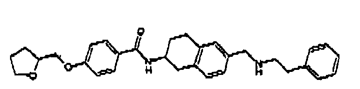 | I144 | 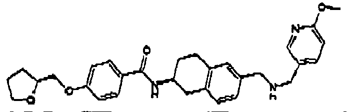 |
| I145 | 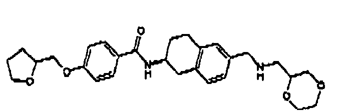 | I157 | 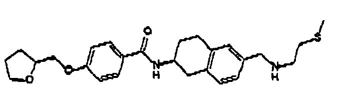 |
| I146 | 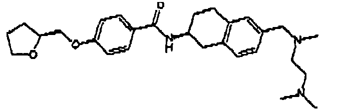 | I158 | 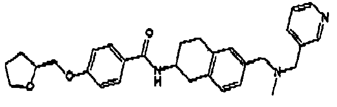 |
| I147 | 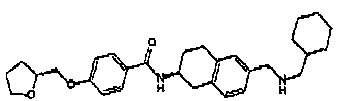 | I159 | 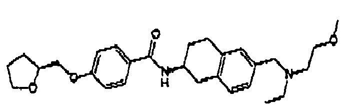 |
| I148 | 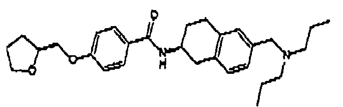 | I160 | 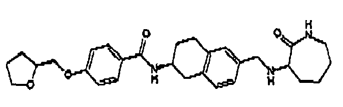 |
| I149 | 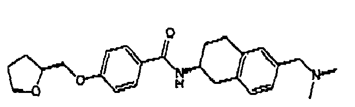 | I161 | 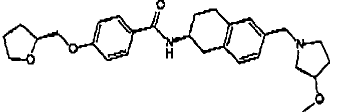 |
| I150 | 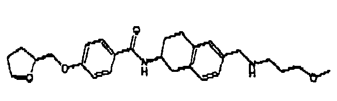 | I162 | 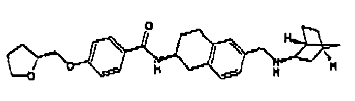 |
| I151 | 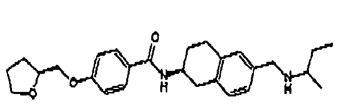 | I163 | 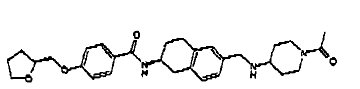 |
| I152 | 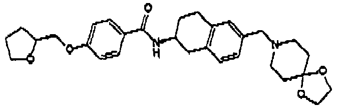 | I164 | 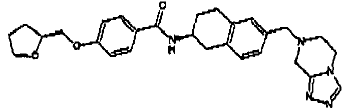 |
| I153 | 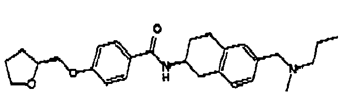 | I165 | 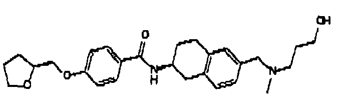 |
| I154 | 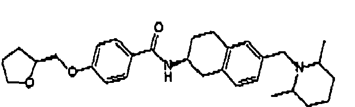 | I166 | 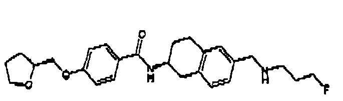 |
| I165 | 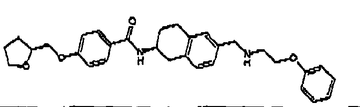 | I167 | 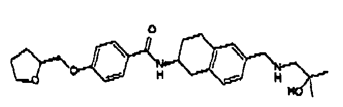 |
| I156 | 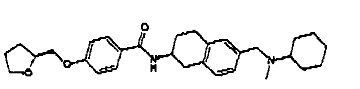 | | |

In Tabelle 2 sind Ergebnisse zusammengefasst, die im oben beschriebenen Kalziummobilisierungsassay erhalten wurden.

**Tabelle 2.**

| Bsp. No. | IC₅₀ / µM |
|---|---|
| 1 | 0,13 |
| 2 | 0,67 |

## Patentansprüche

1. Verbindungen der Formel I 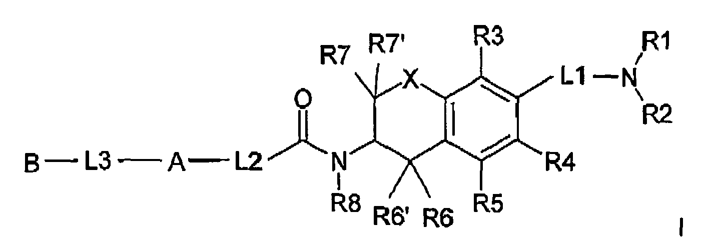 worin bedeuten
R1, R2 unabhängig voneinander H, (C₁-C8)-Alkyl, (C₁-C₄)-Alkoxy-(C₁-C₄)-alkyl, (C₃-C₈)-Alkenyl, (C₃-C₈)-Alkinyl, CO(R9), (C(R10)(R11))_{q}-R12, CO(C(R13)(R14)ᵣ-R15, CO-O(C₁-C₈)-Alkyl, CO(C(R13)(R14))ᵣ-N(R16)(R17);
oder
R1 und R2 bilden zusammen mit dem Stickstoffatom, an das sie gebunden sind, einen 4 bis 10-gliedrigen mono-, bi- oder spirocyclischen Ring welcher außer dem Stickstoffatom 0 bis 3 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, Br, CF₃, CN, (C₁-C₆)-Alkyl, (C₃-C₈)-Cycloalkyl, O-(C₁-C₈)-Alkyl, (C₁-C₄)-Alkoxy-(C₁-C₄)-alkyl, Hydroxy-(C₁-C₆)-alkyl, Oxo, CO(R18), CON(R19)(R20), Hydroxy, COO(R21), N(R22)CO(C₁-C₆)-Alkyl, N(R23)(R24) oder SO₂(C₁-C₆)-Alkyl;
R10, R11 unabhängig voneinander H, (C₁-C₆)-Alkyl, OH;
R9, R13, R14, R18, R19, R20, R21, R22, R23, R24, unabhängig voneinander H, (C₁-C₆)-Alkyl;
oder
R23 und R24 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
q, r unabhängig voneinander 0, 1, 2, 3, 4, 5, 6;
R12, R15 unabhängig voneinander H, OH, F, O-(C₁-C₆)-Alkyl, S-(C₁-C₆)-Alkyl, CN, COO(R25), N(R26)CO(C₁-C₆)-Alkyl, N(R27)(R28), CON(R29)(R30), SO₂(C₁-C₆)-Alkyl, 3-12 gliedriger mono-, bi- oder spirocyclischer Ring, der ein oder mehrere Heteroatome aus der Gruppe N, O und S enthalten kann und der 3-12 gliedrige Ring weitere Substituenten wie F, Cl, Br, OH, CF₃, NO₂, CN, OCF₃, Oxo, O-(C₁-C₆)-Alkyl, (C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, N(R31)(R32), COO(R33), SO₂(C₁-C₆)-Alkyl und COOH enthalten kann;
R25, R26, R27, R28, R29, R30, R31, R32, R33 unabhängig voneinander H, (C₁-C₈)-Alkyl;
oder
R27 und R28, R29 und R30, R31 und R32 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
L1 C(R34)(R35), C(R36)(R37)C(R38)(R39), (C₃-C₆)-Cycloakyl; optional kann R1 mit einem der Reste R34, R35, R36, R37, R38 oder R39 verbunden sein, so dass ein 5-6 gliedriger Ring entsteht;
R34, R35, R36, R37, R38, R39 unabhängig voneinander H, (C₁-C₈)-Alkyl;
R3, R4, R5 unabhängig voneinander H, F, Cl, Br, I, OH, CF₃, NO₂, CN, OCF₃, O-(C₁-C₆)-Alkyl, S-(C₁-C₆)-Alkyl, O-(C₁-C₄)-Alkoxy-(C₁-C₄)-alkyl, (C₁-C₆)-Alkyl, CON(R40)(R41), CO(R42);
R40, R41, R42 unabhängig voneinander H, (C₁-C₈)-Alkyl;
oder
R40 und R41 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
X O, C(R43)(R43');
R6, R6', R7, R7', R43, R43' unabhängig voneinander H, F, (C₁-C₈)-Alkyl, OH, O-(C₁-C₆)-Alkyl;
oder
R6 und R6', oder R43 und R43' zusammen optional Oxo;
R8 H, (C₁-C₈)-Alkyl;
L2 Bindung, C(R44)(R45);
R44, R45 unabhängig voneinander H, (C₁-C₈)-Alkyl;
A 5-6 gliedriger aromatischer Ring, der bis zu 2 Heteroatome ausgewählt aus der Gruppe Stickstoff, Sauerstoff und Schwefel beinhalten kann, und substituiert sein kann mit einem oder mehreren der Substituenten H, F, Cl, Br, I, OH, CF₃, NO₂, CN, OCF₃, O-(C₁-C₆)-Alkyl, O-(C₁-C₄)-Alkoxy-(C₁-C₄)-alkyl, (C₁-C₆)-Alkyl, N(R54)(R55), SO₂-CH₃, CON(R56)(R57), N(R58)CO(B59), CO(R60);
im Fall L2 = Bindung kann C(O)NR8 mit einem ortho-ständigen Substituenten von A über eine Brücke enthaltend ein oder zwei Elemente aus der Gruppe Kohlenstoff und Stickstoff verbunden sein, so dass insgesamt ein 9 bis 10-gliedriger bicyclischer Ring entsteht;
R54, R55, R56, R57, R58, R59, R60 unabhängig voneinander H, (C₁-C₈)-Alkyl;
oder
R54 und R55, R56 und R57 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
L3 Bindung oder ein Linker mit 1 bis 4 Gliedern, wobei die Glieder ausgewählt sind aus der Gruppe bestehend aus O, S, SO₂, N(R61), CO, C(R62)(R63), C≡C, wobei sich ein chemisch sinnvoller Rest ergibt, und der Linker keine Gruppen O-CO oder COO aufweist;
B (C₁-C₆)-Alkyl, (C₁-C₄)-Alkoxy-(C₁-C₄)-alkyl, Hydroxy-(C₁-C₆)-alkyl, ein 3 bis 10-gliedriger mono-, bi- oder spirocyclischer nicht-aromatischer Ring, welcher 0 bis 3 Heteroatome beinhalten kann, ausgewählt aus der Gruppe Sauerstoff, Stickstoff und Schwefel, wobei das Ringsystem zusätzlich substituiert sein kann mit einem oder mehreren der folgenden Substituenten: F, CF₃, (C₁-C₆)-Alkyl, O-(C₁-C₈)-Alkyl, (C₁-C₄)-Alkoxy-(C₁-C₄)-alkyl, Hydroxy-(C₁-C₄)-alkyl, Oxo, CO(R64), Hydroxy;
R61, R62, R63, R64 unabhängig voneinander H, (C₁-C₈)-Alkyl;
wobei im Fall X = C(R43)(R43')
L3 C(R62)(R63)O, und
B ein 4 bis 10-gliedriger mono-, bi- oder spirocyclischer nicht-aromatischer Ring, welcher 1 bis 3 Heteroatome beinhaltet, ausgewählt aus der Gruppe Sauerstoff, Stickstoff und Schwefel, wobei das Ringsystem zusätzlich substituiert sein kann mit einem oder mehreren der folgenden Substituenten: F, CF₃, (C₁-C₆)-Alkyl, O-(C₁-C₈)-Alkyl, (C₁-C₄)-Alkoxy-(C₁-C₄)-alkyl, Hydroxy-(C₁-C₄)-alkyl, Oxo, CO(R64), Hydroxy;
bedeuten;
sowie deren physiologisch verträgliche Salze.

2. Verbindungen der Formel I gemäß Anspruch 1, worin
L2 Bindung bedeutet.

3. Verbindungen der Formel I gemäß Anspruch 1 oder 2, worin
R1, R2 R1 und R2 bilden zusammen mit dem Stickstoffatom, an das sie gebunden sind, einen 4 bis 10-gliedrigen mono-, bi- oder spirocyclischen Ring welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, Br, CF₃, (C₁-C₆)-Alkyl, O-(C₁-C₈)-Alkyl, (C₃-C₈)-Cycloalkyl, (C₁-C₄)-Alkoxy-(C₁-C₄)-alkyl, Hydroxy-(C₁-C₆)-alkyl, Oxo, CO(R18), Hydroxy, N(R22)CO(C₁-C₆)-Alkyl, oder SO₂(C₁-C₆)-Alkyl;
bedeuten.

4. Verbindungen der Formel I gemäß Ansprüchen 1 bis 3, worin
A ausgewählt wird aus der Gruppe 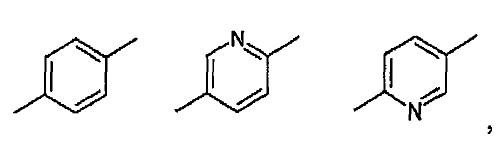
worin A die in Anspruch 1 genannten Substituenten tragen kann.

5. Verbindungen der Formel I gemäß Ansprüchen 1 bis 4, worin
L3 Bindung, O oder C(R62)(R63)O bedeutet

6. Verbindungen der Formel I gemäß Ansprüchen 1 bis 5, worin
B ein 4 bis 6-gliedriger nicht-aromatischer Ring, welcher 1 bis 2 Sauerstoffatome beinhaltet, wobei das Ringsystem zusätzlich substituiert sein kann mit einem oder mehreren der folgenden Substituenten: (C₁-C₆)-Alkyl oder Hydroxy; bedeutet.

7. Verbindungen der Formel I gemäß Ansprüchen 1 bis 6, worin
das kombinierte Element B-L3 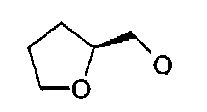 bedeutet.

8. Verbindungen gemäß Anspruch 1, **gekennzeichnet durch** die Formel II 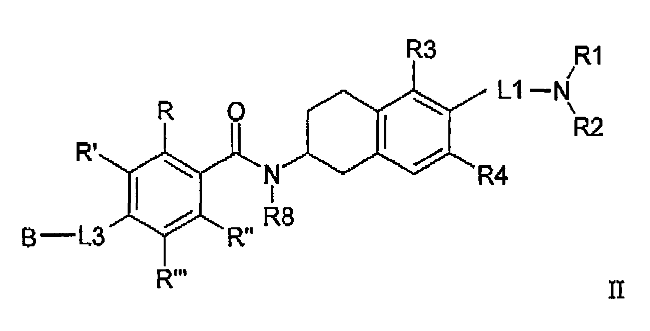 worin die Variablen R1, R2, L1, R3, R4 und R8 die in Anspruch 1 angegebenen Bedeutungen
haben und
R, R', R'', R''' unabhängig voneinander H, F, Cl, Br, I, OH, CF₃, NO₂, CN, OCF₃, O-(C₁-C₆)-Alkyl, O-(C₁-C₄)-Alkoxy-(C₁-C₄)-alkyl, (C₁-C₆)-Alkyl, N(R54)(R55), SO₂-CH₃, CON(R56)(R57), N(R58)CO(R59), CO(R60);
L3 CH₂O;
B 4 bis 6-gliedriger nicht-aromatischer Ring, welcher 1 bis 2 Sauerstoffatome beinhaltet, wobei das Ringsystem zusätzlich substituiert sein kann mit einem oder mehreren der folgenden Substituenten: F, (C₁-C₆)-Alkyl, O-(C₁-C₈)-Alkyl, (C₁-C₄)-Alkoxy-(C₁-C₄)-alkyl, Oxo, Hydroxy bevorzugt (C₁-C₆)-Alkyl oder Hydroxy;
bedeuten.

9. Verbindungen der Formel II, gemäß Anspruch 8, worin
das kombinierte Element B-L3 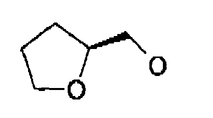 bedeutet.

10. Verbindungen gemäß Anspruch 1, **gekennzeichnet durch** die Formel III 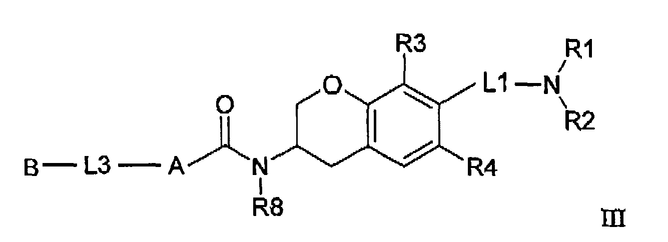 worin R1, R2, R3, R4, R8, A, L1, L3 und B die für Formel I in Anspruch 1 angegebenen Bedeutungen haben.

11. Verbindungen gemäß Anspruch 1, **gekennzeichnet durch** die Formel IV 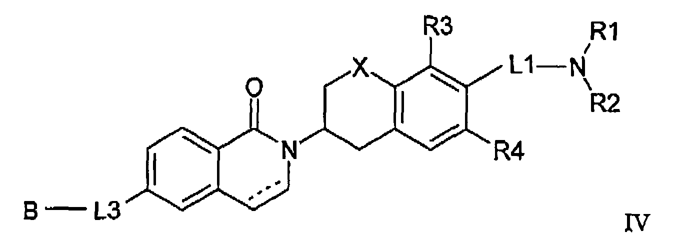 worin R1, R2, R3, R4, X, L1, L3 und B die für Formel I in Anspruch 1 angegebenen Bedeutungen haben, worin die unterbrochene Linie eine optionale Doppelbindung anzeigt, so dass sowohl Dihydroisochinolinone als auch Isochinolinone von der Formel IV umfasst werden.

12. Arzneimittel enthaltend eine oder mehrere der Verbindungen der Formel I gemäß einem oder mehreren der Ansprüche 1 bis 11.

13. Arzneimittel enthaltend eine oder mehrere der Verbindungen der Formel I gemäß einem oder mehreren der Ansprüche 1 bis 11 und einen oder mehrere Wirkstoffe, die günstige Wirkungen auf Stoffwechselstörungen oder damit assoziierte Erkrankungen haben.

14. Arzneimittel enthaltend eine oder mehrere der Verbindungen der Formel I gemäß einem oder mehreren der Ansprüche 1 bis 11 und ein oder mehrere Antidiabetika.

15. Arzneimittel enthaltend eine oder mehrere der Verbindungen der Formel I gemäß einem oder mehreren der Ansprüche 1 bis 11 und einen oder mehrere Lipidmodulatoren.

16. Arzneimittel enthaltend eine oder mehrere der Verbindungen der Formel I gemäß einem oder mehreren der Ansprüche 1 bis 11 und einen oder mehrere Antiadiposita.

17. Verwendung der Verbindungen der Formel I gemäß einem oder mehreren der Ansprüche 1 bis 11 zur Behandlung und/oder Prävention von Störungen des Fettsäurestoffwechsels und Glucoseverwertungsstörungen.

18. Verwendung der Verbindungen der Formel I gemäß einem oder mehreren der Ansprüche 1 bis 11 zur Behandlung und/oder Prävention von Störungen, bei denen Insulinresistenz eine Rolle spielt.

19. Verwendung der Verbindungen der Formel I gemäß einem oder mehreren der Ansprüche 1 bis 11 zur Behandlung und/oder Prävention von Diabetes mellitus und der damit verbundenen Folgeerkrankungen.

20. Verwendung der Verbindungen der Formel I gemäß einem oder mehreren der Ansprüche 1 bis 11 zur Behandlung und/oder Prävention von Dyslipidämien und deren Folgen.

21. Verwendung der Verbindungen der Formel I gemäß einem oder mehreren der Ansprüche 1 bis 11 zur Behandlung und/oder Prävention von nichtalkoholischer Fettleber und ihren Verlaufsformen.

22. Verwendung der Verbindungen der Formel I gemäß einem oder mehreren der Ansprüche 1 bis 11 zur Behandlung und/oder Prävention von Zuständen, die mit dem Metabolischen Syndrom assoziert sind.

23. Verwendung der Verbindungen der Formel I gemäß einem oder mehreren der Ansprüche 1 bis 11 zur Behandlung und/oder Prävention von Obesitas und damit verbundenen Folgeerkrankungen.

24. Verwendung der Verbindungen gemäß einem oder mehreren der Ansprüche 1 bis 11 in Kombination mit mindestens einem weiteren Wirkstoff zur Behandlung und/oder Prävention von Störungen des Fettsäurestoffwechsels und Glucoseverwertungsstörungen.

25. Verwendung der Verbindungen gemäß einem oder mehreren der Ansprüche 1 bis 11 in Kombination mit mindestens einem weiteren Wirkstoff zur Behandlung und/oder Prävention von Störungen, bei denen Insulinresistenz eine Rolle spielt.

26. Verfahren zur Herstellung eines Arzneimittels enthaltend eine oder mehrere der Verbindungen gemäß einem oder mehreren der Ansprüche 1 bis 11, **dadurch gekennzeichnet, dass** der Wirkstoff mit einem pharmazeutisch geeigneten Träger vermischt wird und diese Mischung in eine für die Verabreichung geeignete Form gebracht wird.
